# EUROPEAN PATENT SPECIFICATION

(11) **EP 0 877 803 B1**
(45) Date of publication and mention of the grant of the patent: **31.08.2005**
(21) Application number: 97901343.0
(22) Date of filing: 02.01.1997
(51) Int. Cl.: C12N 15/12, C12N 5/10, C07K 14/715, C07K 16/28, C12Q 1/68, G01N 33/50, A61K 38/17, A61K 48/00

(54) **OB PROTEIN RECEPTOR AND RELATED COMPOSITIONS AND METHODS**
REZEPTOR DES OB-PROTEINS UND VERWANDTE ZUSAMMENSETZUNGEN UND VERFAHREN
RECEPTEUR DE PROTEINE OB, COMPOSITIONS ET PROCEDES ASSOCIES

(30) Priority: 04.01.1996 US 582825; 31.12.1996 US 774414
(43) Date of publication of application: 18.11.1998
(73) Proprietor: Amgen Inc.,, California 91320-1799 (US)
(72) Inventor: CHANG, Ming-Shi, Newbury Park, CA 91320 (US); WELCHER, Andrew, Avery, Thousand Oaks, CA 91320 (US); FLETCHER, Frederick, Addison, Camarillo, CA 93012 (US)
(74) Representative: Brown, John D.
(86) International application number: PCT/US1997/000128
(87) International publication number: WO 1997/025424

(56) References cited:
- WO-A-96/08510
- WO-A-97/19952
- WO-A-97/25425
- WO-A-97/26335
- WO-A-97/27286
- CELL, vol. 83, 29 December 1995, pages 1263-1271, XP000602068 TARTAGLIA,L.A., ET AL . : "IDENTIFICATION AND EXPRESSION CLONING OF A LEPTIN RECEPTOR, OB-R" & EMBL SEQUENCE DATA LIBRARY, 30 December 1995, TARTAGLIA, L.A., ET AL . : "IDENTIFICATION AND EXPRESSION CLONING OF A LEPTIN RECEPTOR, OB-R"
- NATURE MEDICINE, vol. 2, no. 5, May 1996, pages 585-589, XP0002019361 CIOFFI, J.A., ET AL . : "NOVEL B219/OB RECEPTOR ISOFORMS: POSSIBLE ROLE OF LEPTIN IN HEMATOPOIESIS AND REPRODUCTION" & EMBL SEQUENCE DATA LIBRARY, 26 April 1996, HEIDELBERG, GERMANY, CIOFFI, J.A., ET AL . : "NOVEL B219/OB ISOFORMS: POSSIBLE ROLE OF LEPTIN IN HEMATOPOIESIS AND REPRODUCTION"
- CURRENT BIOLOGY, vol. 6, no. 9, 1 September 1996, pages 1170-1180, XP000673008 BENNETT, B.D., ET AL.: "A ROLE FOR LEPTIN AND ITS COGNATE RECEPTOR IN HEMATOPOIESIS" & EMBL SEQUENCE DATA LIBRARY, 7 September 1996, HEIDELBERG, GERMANY, BENNETT, B.D., ET AL . : "A ROLE FOR LEPTIN AND ITS COGNATE RECEPTOR IN HEMATOPOIESIS"
- BIOCHEMICAL AND BIOPHYSICAL RESEARCH COMMUNICATIONS, vol. 224, no. 2, 16 July 1996, pages 597-604, XP002029745 IIDA, M., ET AL . : "SUBSTITUTION AT CODON 269 (GLUTAMINE - PROLIN) OF THE LEPTIN RECEPTOR (OB-R) cDNA IS THE ONLY MUTATION FOUND IN THE ZUCKER FATTY (fa/fa) RAT" & EMBL SEQUENCE DATA LIBRARY, 12 June 1996, HEIDELBERG, GERMANY, IIDA, M., ET AL . : "PHENOTYPE-LINKED AMINO-ACID ALTERATION IN LEPTIN RECEPTOR cDNA FROM ZUCKER FATTY (fa/fa) RAT"
- CELL, vol. 84, 9 February 1996, pages 491-495, XP002029746 CHEN, H., ET AL . : "EVIDENCE THAT THE DIABETES GENE ENCODES THE LEPTIN RECEPTOR: IDENTIFICATION OF A MUTATION IN THE LEPTIN RECEPTOR GENE IN db/db MICE" & EMBL SEQUENCE DATA LIBRARY, 11 February 1996, HEIDELBERG, GERMANY, CHEN,H., ET AL . : "EVIDENCE THAT THE DIABETES GENE ENCODES THE LEPTIN RECEPTOR: IDENTIFICATION OF A MUTATION IN THE LEPTIN RECEPTOR GENE IN db/db MICE"

### SEQUENCE LISTING

(1) GENERAL INFORMATION:
   (i) APPLICANT: CHANG, MING-SHI WELCHER, ANDREW A. FLETCHER, FREDERICK A.
   (ii) TITLE OF INVENTION: OB PROTEIN RECEPTOR AND RELATED COMPOSITIONS AND METHODS
   (iii) NUMBER OF SEQUENCES: 33
   (iv) CORRESPONDENCE ADDRESS:
      (A) ADDRESSEE: Amgen Inc.
      (B) STREET: 1840 Dehavilland Drive
      (C) CITY: Thousand Oaks
      (D) STATE: California
      (E) COUNTRY: USA
      (F) ZIP: 91320
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Pessin, Karol M.
      (C) REFERENCE/DOCKET NUMBER: A-382-A
(2) INFORMATION FOR SEQ ID NO:1:
   (i) SEQUENCE CHARACTERISTICS:
      (A) LENGTH: 965 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:1:
   Met Ile Cys Gln Lys Phe Cys Val Val Leu Leu His Trp Glu Phe Ile
   1 5 10 15
   Tyr Val Ile Thr Ala Phe Asn Leu Ser Tyr Pro Ile Thr Pro Trp Arg
   20 25 30
   Phe Lys Leu Ser Cys Met Pro Pro Asn Ser Thr Tyr Asp Tyr Phe Leu
   35 40 45
   Leu Pro Ala Gly Leu Ser Lys Asn Thr Ser Asn Ser Asn Gly His Tyr
   50 55 60
   Glu Thr Ala Val Glu Pro Lys Phe Asn Ser Ser Gly Thr His Phe Ser
   65 70 75 80
   Asn Leu Ser Lys Thr Thr Phe His Cys Cys Phe Arg Ser Glu Gln Asp
   85 90 95
   Arg Asn Cys Ser Leu Cys Ala Asp Asn Ile Glu Gly Lys Thr Phe Val
   100 105 110
   Ser Thr Val Asn Ser Leu Val Phe Gln Gln Ile Asp Ala Asn Trp Asn
   115 120 125
   Ile Gln Cys Trp Leu Lys Gly Asp Leu Lys Leu Phe Ile Cys Tyr Val
   130 135 140
   Glu Ser Leu Phe Lys Asn Leu Phe Arg Asn Tyr Asn Tyr Lys Val His
   145 150 155 160
   Leu Leu Tyr Val Leu Pro Glu Val Leu Glu Asp Ser Pro Leu Val Pro
   165 170 175
   Gln Lys Gly Ser Phe Gln Met Val His Cys Asn Cys Ser Val His Glu
   180 185 190
   Cys Cys Glu Cys Leu Val Pro Val Pro Thr Ala Lys Leu Asn Asp Thr
   195 200 205
   Leu Leu Met Cys Leu Lys Ile Thr Ser Gly Gly Val Ile Phe Gln Ser
   210 215 220
   Pro Leu Met Ser Val Gln Pro Ile Asn Met Val Lys Pro Asp Pro Pro
   225 230 235 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
           275                 280                 285
   Ser Ala Thr Ser Leu Leu Val Asp Ser Ile Leu Pro Gly Ser Ser Tyr
       290                 295                 300
   Glu Val Gln Val Arg Gly Lys Arg Leu Asp Gly Pro Gly Ile Trp Ser
   305                 310                 315                 320
   Asp Trp Ser Thr Pro Arg Val Phe Thr Thr Gln Asp Val Ile Tyr Phe
                   325                 330                 335
   Pro Pro Lys Ile Leu Thr Ser Val Gly Ser Asn Val Ser Phe His Cys
               340                 345                 350
   Ile Tyr Lys Lys Glu Asn Lys Ile Val Pro Ser Lys Glu Ile Val Trp
           355                 360                 365
   Trp Met Asn Leu Ala Glu Lys Ile Pro Gln Ser Gln Tyr Asp Val Val
       370                 375                 380
   Ser Asp His Val Ser Lys Val Thr Phe Phe Asn Leu Asn Glu Thr Lys
   385                 390                 395                 400
   Pro Arg Gly Lys Phe Thr Tyr Asp Ala Val Tyr Cys Cys Asn Glu His
                   405                 410                 415
   Glu Cys His His Arg Tyr Ala Glu Leu Tyr Val Ile Asp Val Asn Ile
               420                 425                 430
   Asn Ile Ser Cys Glu Thr Asp Gly Tyr Leu Thr Lys Met Thr Cys Arg
           435                 440                 445
   Trp Ser Thr Ser Thr Ile Gln Ser Leu Ala Glu Ser Thr Leu Gln Leu
       450                 455                 460
   Arg Tyr His Arg Ser Ser Leu Tyr Cys Ser Asp Ile Pro Ser Ile His
   465                 470                 475                 480
   Pro Ile Ser Glu Pro Lys Asp Cys Tyr Leu Gln Ser Asp Gly Phe Tyr
                   485                 490                 495
   Glu Cys Ile Phe Gln Pro Ile Phe Leu Leu Ser Gly Tyr Thr Met Trp
               500                 505                 510
   Ile Arg Ile Asn His Ser Leu Gly Ser Leu Asp Ser Pro Pro Thr Cys
           515                 520                 525
   Val Leu Pro Asp Ser Val Val Lys Pro Leu Pro Pro Ser Ser Val Lys
       530                 535                 540
   Ala Glu Ile Thr Ile Asn Ile Gly Leu Leu Lys Ile Ser Trp Glu Lys
   545 550 555 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625 630 635 640
   Arg Gly Pro Glu Phe Trp Arg Ile Ile Asn Gly Asp Thr Met Lys Lys
   645 650 655
   Glu Lys Asn Val Thr Leu Leu Trp Lys Pro Leu Met Lys Asn Asp Ser
   660 665 670
   Leu Cys Ser Val Gln Arg Tyr Val Ile Asn His His Thr Ser Cys Asn
   675 680 685
   Gly Thr Trp Ser Glu Asp Val Gly Asn His Thr Lys Phe Thr Phe Leu
   690 695 700
   Trp Thr Glu Gln Ala His Thr Val Thr Val Leu Ala Ile Asn Ser Ile
   705 710 715 720
   Gly Ala Ser Val Ala Asn Phe Asn Leu Thr Phe Ser Trp Pro Met Ser
   725 730 735
   Lys Val Asn Ile Val Gln Ser Leu Ser Ala Tyr Pro Leu Asn Ser Ser
   740 745 750
   Cys Val Ile Val Ser Trp Ile Leu Ser Pro Ser Asp Tyr Lys Leu Met
   755 760 765
   Tyr Phe Ile Ile Glu Trp Lys Asn Leu Asn Glu Asp Gly Glu Ile Lys
   770 775 780
   Trp Leu Arg Ile Ser Ser Ser Val Lys Lys Tyr Tyr Ile His Asp His
   785 790 795 800
   Phe Ile Pro Ile Glu Lys Tyr Gln Phe Ser Leu Tyr Pro Ile Phe Met
   805 810 815
   Glu Gly Val Gly Lys Pro Lys Ile Ile Asn Ser Phe Thr Gln Asp Asp
   820 825 830
   Ile Glu Lys His Gln Ser Asp Ala Gly Leu Tyr Val Ile Val Pro Val
           835                     840                     845
   Ile Ile Ser Ser Ser Ile Leu Leu Leu Gly Thr Leu Leu Ile Ser His
       850                     855                     860
   Gln Arg Met Lys Lys Leu Phe Trp Glu Asp Val Pro Asn Pro Lys Asn
   865                     870                     875                     880
   Cys Ser Trp Ala Gln Gly Leu Asn Phe Gln Lys Arg Thr Asp Ile Leu
                   885                     890                     895
   Ser Leu Ile Met Ile Thr Thr Asp Glu Pro Asn Val Pro Thr Ser Gln
               900                     905                     910
   Gln Ser Ile Glu Tyr Lys Ile Phe Thr Phe Arg Arg Gly Ala Asn Leu
           915                     920                     925
   Lys Lys Ile Gln Leu Asn Phe Glu Leu Thr Tyr Gly Gly Leu Cys Phe
       930                     935                     940
   Arg Thr Asn Arg Cys Val Asn Leu Gly Ser Lys Cys Arg Phe Glu Ser
   945                     950                     955                     960
   Ser Leu Asp Val Leu
                   965
(2) INFORMATION FOR SEQ ID NO:2:
   (i) SEQUENCE CHARACTERISTICS:
      (A) LENGTH: 3193 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
   CCGCCGCCAT CTCTGCCTTC GGTCGAGTTG GACCCCCGGA TCAAGGTGTA CTTCTCTGAA     60
   GTAAGATGAT TTGTCAAAAA TTCTGTGTGG TTTTGTTACA TTGGGAATTT ATTTATGTGA    120
   TAACTGCGTT TAACTTGTCA TATCCAATTA CTCCTTGGAG ATTTAAGTTG TCTTGCATGC    180
   CACCAAATTC AACCTATGAC TACTTCCTTT TGCCTGCTGG ACTCTCAAAG AATACTTCAA    240
   ATTCGAATGG ACATTATGAG ACAGCTGTTG AACCTAAGTT TAATTCAAGT GGTACTCACT    300
   TTTCTAACTT ATCCAAAACA ACTTTCCACT GTTGCTTTCG GAGTGAGCAA GATAGAAACT 360
   GCTCCTTATG TGCAGACAAC ATTGAAGGAA AGACATTTGT TTCAACAGTA AATTCTTTAG 420
   TTTTTCAACA AATAGATGCA AACTGGAACA TACAGTGCTG GCTAAAAGGA GACTTAAAAT 480
   TATTCATCTG TTATGTGGAG TCATTATTTA AGAATCTATT CAGGAATTAT AACTATAAGG 540
   TCCATCTTTT ATATGTTCTG CCTGAAGTGT TAGAAGATTC ACCTCTGGTT CCCCAAAAAG 600
   GCAGTTTTCA GATGGTTCAC TGCAATTGCA GTGTTCATGA ATGTTGTGAA TGTCTTGTGC 660
   CTGTGCCAAC AGCCAAACTC AACGACACTC TCCTTATGTG TTTGAAAATC ACATCTGGTG 720
   GAGTAATTTT CCAGTCACCT CTAATGTCAG TTCAGCCCAT AAATATGGTG AAGCCTGATC 780
   CACCATTAGG TTTGCATATG GAAATCACAG ATGATGGTAA TTTAAAGATT TCTTGGTCCA 840
   GCCCACCATT GGTACCATTT CCACTTCAAT ATCAAGTGAA ATATTCAGAG AATTCTACAA 900
   CAGTTATCAG AGAAGCTGAC AAGATTGTCT CAGCTACATC CCTGCTAGTA GACAGTATAC 960
   TTCCTGGGTC TTCGTATGAG GTTCAGGTGA GGGGCAAGAG ACTGGATGGC CCAGGAATCT 1020
   GGAGTGACTG GAGTACTCCT CGTGTCTTTA CCACACAAGA TGTCATATAC TTTCCACCTA 1080
   AAATTCTGAC AAGTGTTGGG TCTAATGTTT CTTTTCACTG CATCTATAAG AAGGAAAACA 1140
   AGATTGTTCC CTCAAAAGAG ATTGTTTGGT GGATGAATTT AGCTGAGAAA ATTCCTCAAA 1200
   GCCAGTATGA TGTTGTGAGT GATCATGTTA GCAAAGTTAC TTTTTTCAAT CTGAATGAAA 1260
   CCAAACCTCG AGGAAAGTTT ACCTATGATG CAGTGTACTG CTGCAATGAA CATGAATGCC 1320
   ATCATCGCTA TGCTGAATTA TATGTGATTG ATGTCAATAT CAATATCTCA TGTGAAACTG 1380
   ATGGGTACTT AACTAAAATG ACTTGCAGAT GGTCAACCAG TACAATCCAG TCACTTGCGG 1440
   AAAGCACTTT GCAATTGAGG TATCATAGGA GCAGCCTTTA CTGTTCTGAT ATTCCATCTA 1500
   TTCATCCCAT ATCTGAGCCC AAAGATTGCT ATTTGCAGAG TGATGGTTTT TATGAATGCA 1560
   TTTTCCAGCC AATCTTCCTA TTATCTGGCT ACACAATGTG GATTAGGATC AATCACTCTC 1620
   TAGGTTCACT TGACTCTCCA CCAACATGTG TCCTTCCTGA TTCTGTGGTG AAGCCACTGC 1680
   CTCCATCCAG TGTGAAAGCA GAAATTACTA TAAACATTGG ATTATTGAAA ATATCTTGGG 1740
   AAAAGCCAGT CTTTCCAGAG AATAACCTTC AATTCCAGAT TCGCTATGGT TTAAGTGGAA 1800
   AAGAAGTACA ATGGAAGATG TATGAGGTTT ATGATGCAAA ATCAAAATCT GTCAGTCTCC 1860
   CAGTTCCAGA CTTGTGTGCA GTCTATGCTG TTCAGGTGCG CTGTAAGAGG CTAGATGGAC 1920
   TGGGATATTG GAGTAATTGG AGCAATCCAG CCTACACAGT TGTCATGGAT ATAAAAGTTC 1980
   CTATGAGAGG ACCTGAATTT TGGAGAATAA TTAATGGAGA TACTATGAAA AAGGAGAAAA 2040
   ATGTCACTTT ACTTTGGAAG CCCCTGATGA AAAATGACTC ATTGTGCAGT GTTCAGAGAT 2100
   ATGTGATAAA CCATCATACT TCCTGCAATG GAACATGGTC AGAAGATGTG GGAAATCACA 2160
   CGAAATTCAC TTTCCTGTGG ACAGAGCAAG CACATACTGT TACGGTTCTG GCCATCAATT 2220
   CAATTGGTGC TTCTGTTGCA AATTTTAATT TAACCTTTTC ATGGCCTATG AGCAAAGTAA 2280
   ATATCGTGCA GTCACTCAGT GCTTATCCTT TAAACAGCAG TTGTGTGATT GTTTCCTGGA 2340
   TACTATCACC CAGTGATTAC AAGCTAATGT ATTTTATTAT TGAGTGGAAA AATCTTAATG 2400
   AAGATGGTGA AATAAAATGG CTTAGAATCT CTTCATCTGT TAAGAAGTAT TATATCCATG 2460
   ATCATTTTAT CCCCATTGAG AAGTACCAGT TCAGTCTTTA CCCAATATTT ATGGAAGGAG 2520
   TGGGAAAACC AAAGATAATT AATAGTTTCA CTCAAGATGA TATTGAAAAA CACCAGAGTG 2580
   ATGCAGGTTT ATATGTAATT GTGCCAGTAA TTATTTCCTC TTCCATCTTA TTGCTTGGAA 2640
   CATTATTAAT ATCACACCAA AGAATGAAAA AGCTATTTTG GGAAGATGTT CCGAACCCCA 2700
   AGAATTGTTC CTGGGCACAA GGACTTAATT TTCAGAAGAG AACGGACATT CTTTGAAGTC 2760
   TAATCATGAT CACTACAGAT GAACCCAATG TGCCAACTTC CCAACAGTCT ATAGAGTATT 2820
   AGAAGATTTT TACATTTTGA AGAAGGGGAG CAAATCTAAA AAAAATTCAG TTGAACTTCT 2880
   GAGAGTTAAC ATATGGTGGA TTATGTTGAT TTAGAACTTA AAATAGATGT GTAAATTTGG 2940
   GTTCAAAATG TAGATTTGAG TCCAGTTTGG ATGTGTGATT AATTTTCAAA TCATCTAAAG 3000
   TTTAAAAGTA GTATTCATGA TTTCTGGCTT TTGATTTGCC ATATTCCTGG TCATAAAACA 3060
   TTAAGAAAAT TATGGCTGTT GCTGTCATTA CATATCTATT AAATGTCATC AAATATGTAG 3120
   TAGACAATTT TGTAATTAGG TGAACTCTAA AACTGCAACA TCTGACAAAT TGCTTTAAAA 3180
   ATACAATGAT TAT 3193
(2) INFORMATION FOR SEQ ID NO:3:
   (i) SEQUENCE CHARACTERISTICS:
      (A) LENGTH: 995 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:3:
   Met Ile Cys Gln Lys Phe Cys Val Val Leu Leu His Trp Glu Phe Ile
   1               5                   10                  15
   Tyr Val Ile Thr Ala Phe Asn Leu Ser Tyr Pro Ile Thr Pro Trp Arg
               20                  25                  30
   Phe Lys Leu Ser Cys Met Pro Pro Asn Ser Thr Tyr Asp Tyr Phe Leu
           35                  40                  45
   Leu Pro Ala Gly Leu Ser Lys Asn Thr Ser Asn Ser Asn Gly His Tyr
       50                  55                  60
   Glu Thr Ala Val Glu Pro Lys Phe Asn Ser Ser Gly Thr His Phe Ser
   65                  70                  75                  80
   Asn Leu Ser Lys Thr Thr Phe His Cys Cys Phe Arg Ser Glu Gln Asp
                   85                  90                  95
   Arg Asn Cys Ser Leu Cys Ala Asp Asn Ile Glu Gly Lys Thr Phe Val
               100                 105                 110
   Ser Thr Val Asn Ser Leu Val Phe Gln Gln Ile Asp Ala Asn Trp Asn
           115                 120                 125
   Ile Gln Cys Trp Leu Lys Gly Asp Leu Lys Leu Phe Ile Cys Tyr Val
       130                 135                 140
   Glu Ser Leu Phe Lys Asn Leu Phe Arg Asn Tyr Asn Tyr Lys Val His
   145                 150                 155                 160
   Leu Leu Tyr Val Leu Pro Glu Val Leu Glu Asp Ser Pro Leu Val Pro
                   165                 170                 175
   Gln Lys Gly Ser Phe Gln Met Val His Cys Asn Cys Ser Val His Glu
               180                 185                 190
   Cys Cys Glu Cys Leu Val Pro Val Pro Thr Ala Lys Leu Asn Asp Thr
           195                 200                 205
   Leu Leu Met Cys Leu Lys Ile Thr Ser Gly Gly Val Ile Phe Gln Ser
       210                 215                 220
   Pro Leu Met Ser Val Gln Pro Ile Asn Met Val Lys Pro Asp Pro Pro
   225                 230                 235                 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
   275 280 285
   Ser Ala Thr Ser Leu Leu Val Asp Ser Ile Leu Pro Gly Ser Ser Tyr
   290 295 300
   Glu Val Gln Val Arg Gly Lys Arg Leu Asp Gly Pro Gly Ile Trp Ser
   305 310 315 320
   Asp Trp Ser Thr Pro Arg Val Phe Thr Thr Gln Asp Val Ile Tyr Phe
   325 330 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465 470 475 480
   Pro Ile Ser Glu Pro Lys Asp Cys Tyr Leu Gln Ser Asp Gly Phe Tyr
   485 490 495
   Glu Cys Ile Phe Gln Pro Ile Phe Leu Leu Ser Gly Tyr Thr Met Trp
   500 505 510
   Ile Arg Ile Asn His Ser Leu Gly Ser Leu Asp Ser Pro Pro Thr Cys
   515 520 525
   Val Leu Pro Asp Ser Val Val Lys Pro Leu Pro Pro Ser Ser Val Lys
   530 535 540
   Ala Glu Ile Thr Ile Asn Ile Gly Leu Leu Lys Ile Ser Trp Glu Lys
   545 550 555 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625 630 635 640
   Arg Gly Pro Glu Phe Trp Arg Ile Ile Asn Gly Asp Thr Met Lys Lys
   645 650 655
   Glu Lys Asn Val Thr Leu Leu Trp Lys Pro Leu Met Lys Asn Asp Ser
   660 665 670
   Leu Cys Ser Val Gln Arg Tyr Val Ile Asn His His Thr Ser Cys Asn
   675 680 685
   Gly Thr Trp Ser Glu Asp Val Gly Asn His Thr Lys Phe Thr Phe Leu
   690 695 700
   Trp Thr Glu Gln Ala His Thr Val Thr Val Leu Ala Ile Asn Ser Ile
   705 710 715 720
   Gly Ala Ser Val Ala Asn Phe Asn Leu Thr Phe Ser Trp Pro Met Ser
   725 730 735
   Lys Val Asn Ile Val Gln Ser Leu Ser Ala Tyr Pro Leu Asn Ser Ser
   740 745 750
   Cys Val Ile Val Ser Trp Ile Leu Ser Pro Ser Asp Tyr Lys Leu Met
   755 760 765
   Tyr Phe Ile Ile Glu Trp Lys Asn Leu Asn Glu Asp Gly Glu Ile Lys
   770 775 780
   Trp Leu Arg Ile Ser Ser Ser Val Lys Lys Tyr Tyr Ile His Asp His
   785 790 795 800
   Phe Ile Pro Ile Glu Lys Tyr Gln Phe Ser Leu Tyr Pro Ile Phe Met
                   805                 810                 815
   Glu Gly Val Gly Lys Pro Lys Ile Ile Asn Ser Phe Thr Gln Asp Asp
               820                 825                 830
   Ile Glu Lys His Gln Ser Asp Ala Gly Leu Tyr Val Ile Val Pro Val
           835                 840                 845
   Ile Ile Ser Ser Ser Ile Leu Leu Leu Gly Thr Leu Leu Ile Ser His
       850                 855                 860
   Gln Arg Met Lys Lys Leu Phe Trp Glu Asp Val Pro Asn Pro Lys Asn
   865                 870                 875                 880
   Cys Ser Trp Ala Gln Gly Leu Asn Phe Gln Lys Lys Arg Leu Ser Ile
                   885                 890                 895
   Phe Leu Ser Ser Ile Gln His Gln His Val Val Leu Phe Phe Trp Ser
               900                 905                 910
   Leu Lys Gln Phe Gln Lys Ile Ser Val Leu Ile His His Gly Lys Ile
           915                 920                 925
   Lys Met Arg Cys Gln Gln Leu Trp Ser Leu Tyr Phe Gln Gln Gln Ile
       930                 935                 940
   Leu Lys Arg Val Leu Phe Val Leu Val Thr Ser Ser Thr Val Leu Thr
   945                 950                 955                 960
   Ser Leu Arg Leu Arg Val Leu Arg Pro Met Arg Thr Lys Ala Arg Asp
                   965                 970                 975
   Asn Pro Leu Leu Asn Thr Pro Arg Ser Ala Thr Leu Asn Gln Val Lys
               980                 985                 990
   Leu Val Lys
           995
(2) INFORMATION FOR SEQ ID NO:4:
   (i) SEQUENCE CHARACTERISTICS:
      (A) LENGTH: 3063 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
   CCGCCGCCAT CTCTGCCTTC GGTCGAGTTG GACCCCCGGA TCAAGGTGTA CTTCTCTGAA     60
   GTAAGATGAT TTGTCAAAAA TTCTGTGTGG TTTTGTTACA TTGGGAATTT ATTTATGTGA    120
   TAACTGCGTT TAACTTGTCA TATCCAATTA CTCCTTGGAG ATTTAAGTTG TCTTGCATGC    180
   CACCAAATTC AACCTATGAC TACTTCCTTT TGCCTGCTGG ACTCTCAAAG AATACTTCAA    240
   ATTCGAATGG ACATTATGAG ACAGCTGTTG AACCTAAGTT TAATTCAAGT GGTACTCACT    300
   TTTCTAACTT ATCCAAAACA ACTTTCCACT GTTGCTTTCG GAGTGAGCAA GATAGAAACT    360
   GCTCCTTATG TGCAGACAAC ATTGAAGGAA AGACATTTGT TTCAACAGTA AATTCTTTAG    420
   TTTTTCAACA AATAGATGCA AACTGGAACA TACAGTGCTG GCTAAAAGGA GACTTAAAAT    480
   TATTCATCTG TTATGTGGAG TCATTATTTA AGAATCTATT CAGGAATTAT AACTATAAGG    540
   TCCATCTTTT ATATGTTCTG CCTGAAGTGT TAGAAGATTC ACCTCTGGTT CCCCAAAAAG    600
   GCAGTTTTCA GATGGTTCAC TGCAATTGCA GTGTTCATGA ATGTTGTGAA TGTCTTGTGC    660
   CTGTGCCAAC AGCCAAACTC AACGACACTC TCCTTATGTG TTTGAAAATC ACATCTGGTG    720
   GAGTAATTTT CCAGTCACCT CTAATGTCAG TTCAGCCCAT AAATATGGTG AAGCCTGATC    780
   CACCATTAGG TTTGCATATG GAAATCACAG ATGATGGTAA TTTAAAGATT TCTTGGTCCA    840
   GCCCACCATT GGTACCATTT CCACTTCAAT ATCAAGTGAA ATATTCAGAG AATTCTACAA    900
   CAGTTATCAG AGAAGCTGAC AAGATTGTCT CAGCTACATC CCTGCTAGTA GACAGTATAC    960
   TTCCTGGGTC TTCGTATGAG GTTCAGGTGA GGGGCAAGAG ACTGGATGGC CCAGGAATCT   1020
   GGAGTGACTG GAGTACTCCT CGTGTCTTTA CCACACAAGA TGTCATATAC TTTCCACCTA   1080
   AAATTCTGAC AAGTGTTGGG TCTAATGTTT CTTTTCACTG CATCTATAAG AAGGAAAACA   1140
   AGATTGTTCC CTCAAAAGAG ATTGTTTGGT GGATGAATTT AGCTGAGAAA ATTCCTCAAA   1200
   GCCAGTATGA TGTTGTGAGT GATCATGTTA GCAAAGTTAC TTTTTTCAAT CTGAATGAAA   1260
   CCAAACCTCG AGGAAAGTTT ACCTATGATG CAGTGTACTG CTGCAATGAA CATGAATGCC   1320
   ATCATCGCTA TGCTGAATTA TATGTGATTG ATGTCAATAT CAATATCTCA TGTGAAACTG   1380
   ATGGGTACTT AACTAAAATG ACTTGCAGAT GGTCAACCAG TACAATCCAG TCACTTGCGG   1440
   AAAGCACTTT GCAATTGAGG TATCATAGGA GCAGCCTTTA CTGTTCTGAT ATTCCATCTA   1500
   TTCATCCCAT ATCTGAGCCC AAAGATTGCT ATTTGCAGAG TGATGGTTTT TATGAATGCA   1560
   TTTTCCAGCC AATCTTCCTA TTATCTGGCT ACACAATGTG GATTAGGATC AATCACTCTC 1620
   TAGGTTCACT TGACTCTCCA CCAACATGTG TCCTTCCTGA TTCTGTGGTG AAGCCACTGC 1680
   CTCCATCCAG TGTGAAAGCA GAAATTACTA TAAACATTGG ATTATTGAAA ATATCTTGGG 1740
   AAAAGCCAGT CTTTCCAGAG AATAACCTTC AATTCCAGAT TCGCTATGGT TTAAGTGGAA 1800
   AAGAAGTACA ATGGAAGATG TATGAGGTTT ATGATGCAAA ATCAAAATCT GTCAGTCTCC 1860
   CAGTTCCAGA CTTGTGTGCA GTCTATGCTG TTCAGGTGCG CTGTAAGAGG CTAGATGGAC 1920
   TGGGATATTG GAGTAATTGG AGCAATCCAG CCTACACAGT TGTCATGGAT ATAAAAGTTC 1980
   CTATGAGAGG ACCTGAATTT TGGAGAATAA TTAATGGAGA TACTATGAAA AAGGAGAAAA 2040
   ATGTCACTTT ACTTTGGAAG CCCCTGATGA AAAATGACTC ATTGTGCAGT GTTCAGAGAT 2100
   ATGTGATAAA CCATCATACT TCCTGCAATG GAACATGGTC AGAAGATGTG GGAAATCACA 2160
   CGAAATTCAC TTTCCTGTGG ACAGAGCAAG CACATACTGT TACGGTTCTG GCCATCAATT 2220
   CAATTGGTGC TTCTGTTGCA AATTTTAATT TAACCTTTTC ATGGCCTATG AGCAAAGTAA 2280
   ATATCGTGCA GTCACTCAGT GCTTATCCTT TAAACAGCAG TTGTGTGATT GTTTCCTGGA 2340
   TACTATCACC CAGTGATTAC AAGCTAATGT ATTTTATTAT TGAGTGGAAA AATCTTAATG 2400
   AAGATGGTGA AATAAAATGG CTTAGAATCT CTTCATCTGT TAAGAAGTAT TATATCCATG 2460
   ATCATTTTAT CCCCATTGAG AAGTACCAGT TCAGTCTTTA CCCAATATTT ATGGAAGGAG 2520
   TGGGAAAACC AAAGATAATT AATAGTTTCA CTCAAGATGA TATTGAAAAA CACCAGAGTG 2580
   ATGCAGGTTT ATATGTAATT GTGCCAGTAA TTATTTCCTC TTCCATCTTA TTGCTTGGAA 2640
   CATTATTAAT ATCACACCAA AGAATGAAAA AGCTATTTTG GGAAGATGTT CCGAACCCCA 2700
   AGAATTGTTC CTGGGCACAA GGACTTAATT TTCAGAAGAA ACGTTTGAGC ATCTTTTTAT 2760
   CAAGCATACA GCATCAGTGA CATGTGGTCC TCTTCTTTTG GAGCCTGAAA CAATTTCAGA 2820
   AGATATCAGT GTTGATACAT CATGGAAAAA TAAAGATGAG ATGATGCCAA CAACTGTGGT 2880
   CTCTCTACTT TCAACAACAG ATCTTGAAAA GGGTTCTGTT TGTTTTAGTG ACCAGTTCAA 2940
   CAGTGTTAAC TTCTCTGAGG CTGAGGGTAC TGAGGTAACC TATGAGGACG AAAGCCAGAG 3000
   ACAACCCTTT GTTAAATACG CCACGCTGAT CAGCAACTCT AAACCAAGTG AAACTGGTGA 3060
   AGA 3063
(2) INFORMATION FOR SEQ ID NO:5:
   (i) SEQUENCE CHARACTERISTICS:
      (A) LENGTH: 969 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:5:
   Met Ile Cys Gln Lys Phe Cys Val Val Leu Leu His Trp Glu Phe Ile
   1               5                   10                  15
   Tyr Val Ile Thr Ala Phe Asn Leu Ser Tyr Pro Ile Thr Pro Trp Arg
               20                  25                  30
   Phe Lys Leu Ser Cys Met Pro Pro Asn Ser Thr Tyr Asp Tyr Phe Leu
           35                  40                  45
   Leu Pro Ala Gly Leu Ser Lys Asn Thr Ser Asn Ser Asn Gly His Tyr
       50                  55                  60
   Glu Thr Ala Val Glu Pro Lys Phe Asn Ser Ser Gly Thr His Phe Ser
   65                  70                  75                  80
   Asn Leu Ser Lys Thr Thr Phe His Cys Cys Phe Arg Ser Glu Gln Asp
                   85                  90                  95
   Arg Asn Cys Ser Leu Cys Ala Asp Asn Ile Glu Gly Lys Thr Phe Val
               100                 105                 110
   Ser Thr Val Asn Ser Leu Val Phe Gln Gln Ile Asp Ala Asn Trp Asn
           115                 120                 125
   Ile Gln Cys Trp Leu Lys Gly Asp Leu Lys Leu Phe Ile Cys Tyr Val
       130                 135                 140
   Glu Ser Leu Phe Lys Asn Leu Phe Arg Asn Tyr Asn Tyr Lys Val His
   145                 150                 155                 160
   Leu Leu Tyr Val Leu Pro Glu Val Leu Glu Asp Ser Pro Leu Val Pro
                   165                 170                 175
   Gln Lys Gly Ser Phe Gln Met Val His Cys Asn Cys Ser Val His Glu
               180                 185                 190
   Cys Cys Glu Cys Leu Val Pro Val Pro Thr Ala Lys Leu Asn Asp Thr
           195                 200                 205
   Leu Leu Met Cys Leu Lys Ile Thr Ser Gly Gly Val Ile Phe Gln Ser
   210 215 220
   Pro Leu Met Ser Val Gln Pro Ile Asn Met Val Lys Pro Asp Pro Pro
   225 230 235 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
   275 280 285
   Ser Ala Thr Ser Leu Leu Val Asp Ser Ile Leu Pro Gly Ser Ser Tyr
   290 295 300
   Glu Val Gln Val Arg Gly Lys Arg Leu Asp Gly Pro Gly Ile Trp Ser
   305 310 315 320
   Asp Trp Ser Thr Pro Arg Val Phe Thr Thr Gln Asp Val Ile Tyr Phe
   325 330 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465 470 475 480
   Pro Ile Ser Glu Pro Lys Asp Cys Tyr Leu Gln Ser Asp Gly Phe Tyr
                   485                 490                 495
   Glu Cys Ile Phe Gln Pro Ile Phe Leu Leu Ser Gly Tyr Thr Met Trp
               500                 505                 510
   Ile Arg Ile Asn His Ser Leu Gly Ser Leu Asp Ser Pro Pro Thr Cys
           515                 520                 525
   Val Leu Pro Asp Ser Val Val Lys Pro Leu Pro Pro Ser Ser Val Lys
       530                 535                 540
   Ala Glu Ile Thr Ile Asn Ile Gly Leu Leu Lys Ile Ser Trp Glu Lys
   545                 550                 555                 560
   Pro Val Phe Pro Glu Asn Asn Leu Gln Phe Gln Ile Arg Tyr Gly Leu
                   565                 570                 575
   Ser Gly Lys Glu Val Gln Trp Lys Met Tyr Glu Val Tyr Asp Ala Lys
               580                 585                 590
   Ser Lys Ser Val Ser Leu Pro Val Pro Asp Leu Cys Ala Val Tyr Ala
           595                 600                 605
   Val Gln Val Arg Cys Lys Arg Leu Asp Gly Leu Gly Tyr Trp Ser Asn
       610                 615                 620
   Trp Ser Asn Pro Ala Tyr Thr Val Val Met Asp Ile Lys Val Pro Met
   625                 630                 635                 640
   Arg Gly Pro Glu Phe Trp Arg Ile Ile Asn Gly Asp Thr Met Lys Lys
                   645                 650                 655
   Glu Lys Asn Val Thr Leu Leu Trp Lys Pro Leu Met Lys Asn Asp Ser
               660                 665                 670
   Leu Cys Ser Val Gln Arg Tyr Val Ile Asn His His Thr Ser Cys Asn
           675                 680                 685
   Gly Thr Trp Ser Glu Asp Val Gly Asn His Thr Lys Phe Thr Phe Leu
       690                 695                 700
   Trp Thr Glu Gln Ala His Thr Val Thr Val Leu Ala Ile Asn Ser Ile
   705                 710                 715                 720
   Gly Ala Ser Val Ala Asn Phe Asn Leu Thr Phe Ser Trp Pro Met Ser
                   725                 730                 735
   Lys Val Asn Ile Val Gln Ser Leu Ser Ala Tyr Pro Leu Asn Ser Ser
               740                 745                 750
   Cys Val Ile Val Ser Trp Ile Leu Ser Pro Ser Asp Tyr Lys Leu Met
           755                 760                 765
   Tyr Phe Ile Ile Glu Trp Lys Asn Leu Asn Glu Asp Gly Glu Ile Lys
       770                 775                 780
   Trp Leu Arg Ile Ser Ser Ser Val Lys Lys Tyr Tyr Ile His Asp His
   785                 790                 795                 800
   Phe Ile Pro Ile Glu Lys Tyr Gln Phe Ser Leu Tyr Pro Ile Phe Met
                   805                 810                 815
   Glu Gly Val Gly Lys Pro Lys Ile Ile Asn Ser Phe Thr Gln Asp Asp
               820                 825                 830
   Ile Glu Lys His Gln Ser Asp Ala Gly Leu Tyr Val Ile Val Pro Val
           835                 840                 845
   Ile Ile Ser Ser Ser Ile Leu Leu Leu Gly Thr Leu Leu Ile Ser His
       850                 855                 860
   Gln Arg Met Lys Lys Leu Phe Trp Glu Asp Val Pro Asn Pro Lys Asn
   865                 870                 875                 880
   Cys Ser Trp Ala Gln Gly Leu Asn Phe Gln Lys Met Leu Glu Gly Ser
                   885                 890                 895
   Met Phe Val Lys Ser His His His Ser Leu Ile Ser Ser Thr Gln Gly
               900                 905                 910
   His Lys His Cys Gly Arg Pro Gln Gly Pro Leu His Arg Lys Thr Arg
           915                 920                 925
   Asp Leu Cys Ser Leu Val Tyr Leu Leu Thr Leu Pro Pro Leu Leu Ser
       930                 935                 940
   Tyr Asp Pro Ala Lys Ser Pro Ser Val Arg Asn Thr Gln Glu Ser Ile
   945                 950                 955                 960
   Lys Lys Lys Lys Lys Lys Leu Glu Gly
                   965
(2) INFORMATION FOR SEQ ID NO:6:
   (i) SEQUENCE CHARACTERISTICS:
      (A) LENGTH: 969 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:6:
   Met Ile Cys Gln Lys Phe Cys Val Val Leu Leu His Trp Glu Phe Ile
   1               5                   10                  15
   Tyr Val Ile Thr Ala Phe Asn Leu Ser Tyr Pro Ile Thr Pro Trp Arg
               20                  25                  30
   Phe Lys Leu Ser Cys Met Pro Pro Asn Ser Thr Tyr Asp Tyr Phe Leu
           35                  40                  45
   Leu Pro Ala Gly Leu Ser Lys Asn Thr Ser Asn Ser Asn Gly His Tyr
       50                  55                  60
   Glu Thr Ala Val Glu Pro Lys Phe Asn Ser Ser Gly Thr His Phe Ser
   65                  70                  75                  80
   Asn Leu Ser Lys Thr Thr Phe His Cys Cys Phe Arg Ser Glu Gln Asp
                   85                  90                  95
   Arg Asn Cys Ser Leu Cys Ala Asp Asn Ile Glu Gly Lys Thr Phe Val
               100                 105                 110
   Ser Thr Val Asn Ser Leu Val Phe Gln Gln Ile Asp Ala Asn Trp Asn
           115                 120                 125
   Ile Gln Cys Trp Leu Lys Gly Asp Leu Lys Leu Phe Ile Cys Tyr Val
       130                 135                 140
   Glu Ser Leu Phe Lys Asn Leu Phe Arg Asn Tyr Asn Tyr Lys Val His
   145                 150                 155                 160
   Leu Leu Tyr Val Leu Pro Glu Val Leu Glu Asp Ser Pro Leu Val Pro
                   165                 170                 175
   Gln Lys Gly Ser Phe Gln Met Val His Cys Asn Cys Ser Val His Glu
               180                 185                 190
   Cys Cys Glu Cys Leu Val Pro Val Pro Thr Ala Lys Leu Asn Asp Thr
           195                 200                 205
   Leu Leu Met Cys Leu Lys Ile Thr Ser Gly Gly Val Ile Phe Gln Ser
       210                 215                 220
   Pro Leu Met Ser Val Gln Pro Ile Asn Met Val Lys Pro Asp Pro Pro
   225                 230                 235                 240
   Leu Gly Leu His Met Glu Ile Thr Asp Asp Gly Asn Leu Lys Ile Ser
                   245                 250                 255
   Trp Ser Ser Pro Pro Leu Val Pro Phe Pro Leu Gln Tyr Gln Val Lys
               260                 265                 270
   Tyr Ser Glu Asn Ser Thr Thr Val Ile Arg Glu Ala Asp Lys Ile Val
           275                 280                 285
   Ser Ala Thr Ser Leu Leu Val Asp Ser Ile Leu Pro Gly Ser Ser Tyr
       290                 295                 300
   Glu Val Gln Val Arg Gly Lys Arg Leu Asp Gly Pro Gly Ile Trp Ser
   305                 310                 315                 320
   Asp Trp Ser Thr Pro Arg Val Phe Thr Thr Gln Asp Val Ile Tyr Phe
                   325                 330                 335
   Pro Pro Lys Ile Leu Thr Ser Val Gly Ser Asn Val Ser Phe His Cys
               340                 345                 350
   Ile Tyr Lys Lys Glu Asn Lys Ile Val Pro Ser Lys Glu Ile Val Trp
           355                 360                 365
   Trp Met Asn Leu Ala Glu Lys Ile Pro Gln Ser Gln Tyr Asp Val Val
       370                 375                 380
   Ser Asp His Val Ser Lys Val Thr Phe Phe Asn Leu Asn Glu Thr Lys
   385                 390                 395                 400
   Pro Arg Gly Lys Phe Thr Tyr Asp Ala Val Tyr Cys Cys Asn Glu His
                   405                 410                 415
   Glu Cys His His Arg Tyr Ala Glu Leu Tyr Val Ile Asp Val Asn Ile
               420                 425                 430
   Asn Ile Ser Cys Glu Thr Asp Gly Tyr Leu Thr Lys Met Thr Cys Arg
           435                 440                 445
   Trp Ser Thr Ser Thr Ile Gln Ser Leu Ala Glu Ser Thr Leu Gln Leu
       450                 455                 460
   Arg Tyr His Arg Ser Ser Leu Tyr Cys Ser Asp Ile Pro Ser Ile His
   465                 470                 475                 480
   Pro Ile Ser Glu Pro Lys Asp Cys Tyr Leu Gln Ser Asp Gly Phe Tyr
                   485                 490                 495
   Glu Cys Ile Phe Gln Pro Ile Phe Leu Leu Ser Gly Tyr Thr Met Trp
               500                 505                 510
   Ile Arg Ile Asn His Ser Leu Gly Ser Leu Asp Ser Pro Pro Thr Cys
           515                 520                 525
   Val Leu Pro Asp Ser Val Val Lys Pro Leu Pro Pro Ser Ser Val Lys
       530                 535                 540
   Ala Glu Ile Thr Ile Asn Ile Gly Leu Leu Lys Ile Ser Trp Glu Lys
   545                 550                 555                 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625 630 635 640
   Arg Gly Pro Glu Phe Trp Arg Ile Ile Asn Gly Asp Thr Met Lys Lys
   645 650 655
   Glu Lys Asn Val Thr Leu Leu Trp Lys Pro Leu Met Lys Asn Asp Ser
   660 665 670
   Leu Cys Ser Val Gln Arg Tyr Val Ile Asn His His Thr Ser Cys Asn
   675 680 685
   Gly Thr Trp Ser Glu Asp Val Gly Asn His Thr Lys Phe Thr Phe Leu
   690 695 700
   Trp Thr Glu Gln Ala His Thr Val Thr Val Leu Ala Ile Asn Ser Ile
   705 710 715 720
   Gly Ala Ser Val Ala Asn Phe Asn Leu Thr Phe Ser Trp Pro Met Ser
   725 730 735
   Lys Val Asn Ile Val Gln Ser Leu Ser Ala Tyr Pro Leu Asn Ser Ser
   740 745 750
   Cys Val Ile Val Ser Trp Ile Leu Ser Pro Ser Asp Tyr Lys Leu Met
   755 760 765
   Tyr Phe Ile Ile Glu Trp Lys Asn Leu Asn Glu Asp Gly Glu Ile Lys
   770 775 780
   Trp Leu Arg Ile Ser Ser Ser Val Lys Lys Tyr Tyr Ile His Asp His
   785 790 795 800
   Phe Ile Pro Ile Glu Lys Tyr Gln Phe Ser Leu Tyr Pro Ile Phe Met
   805 810 815
   Glu Gly Val Gly Lys Pro Lys Ile Ile Asn Ser Phe Thr Gln Asp Asp
   820 825 830
   Ile Glu Lys His Gln Ser Asp Ala Gly Leu Tyr Val Ile Val Pro Val
   835 840 845
   Ile Ile Ser Ser Ser Ile Leu Leu Leu Gly Thr Leu Leu Ile Ser His
   850 855 860
   Gln Arg Met Lys Lys Leu Phe Trp Glu Asp Val Pro Asn Pro Lys Asn
   865 870 875 880
   Cys Ser Trp Ala Gln Gly Leu Asn Phe Gln Lys Met Leu Glu Gly Ser
   885 890 895
   Met Phe Val Lys Ser His His His Ser Leu Ile Ser Ser Thr Gln Gly
   900 905 910
   His Lys His Cys Gly Arg Pro Gln Gly Pro Leu His Arg Lys Thr Arg
   915 920 925
   Asp Leu Cys Ser Leu Val Tyr Leu Leu Thr Leu Pro Pro Leu Leu Ser
   930 935 940
   Tyr Asp Pro Ala Lys Ser Pro Ser Val Arg Asn Thr Gln Glu Ser Ile
   945 950 955 960
   Lys Lys Lys Lys Lys Lys Leu Glu Gly
   965
(2) INFORMATION FOR SEQ ID NO:7:
   (i) SEQUENCE CHARACTERISTICS:
      (A) LENGTH: 1216 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:7:
   Met Ile Cys Gln Lys Phe Cys Val Val Leu Leu His Trp Glu Phe Ile
   1 5 10 15
   Tyr Val Ile Thr Ala Phe Asn Leu Ser Tyr Pro Ile Thr Pro Trp Arg
   20 25 30
   Phe Lys Leu Ser Cys Met Pro Pro Asn Ser Thr Tyr Asp Tyr Phe Leu
   35 40 45
   Leu Pro Ala Gly Leu Ser Lys Asn Thr Ser Asn Ser Asn Gly His Tyr
   50 55 60
   Glu Thr Ala Val Glu Pro Lys Phe Asn Ser Ser Gly Thr His Phe Ser
   65                  70                  75                  80
   Asn Leu Ser Lys Thr Thr Phe His Cys Cys Phe Arg Ser Glu Gln Asp
                   85                  90                  95
   Arg Asn Cys Ser Leu Cys Ala Asp Asn Ile Glu Gly Lys Thr Phe Val
               100                 105                 110
   Ser Thr Val Asn Ser Leu Val Phe Gln Gln Ile Asp Ala Asn Trp Asn
           115                 120                 125
   Ile Gln Cys Trp Leu Lys Gly Asp Leu Lys Leu Phe Ile Cys Tyr Val
       130                 135                 140
   Glu Ser Leu Phe Lys Asn Leu Phe Arg Asn Tyr Asn Tyr Lys Val His
   145                 150                 155                 160
   Leu Leu Tyr Val Leu Pro Glu Val Leu Glu Asp Ser Pro Leu Val Pro
                   165                 170                 175
   Gln Lys Gly Ser Phe Gln Met Val His Cys Asn Cys Ser Val His Glu
               180                 185                 190
   Cys Cys Glu Cys Leu Val Pro Val Pro Thr Ala Lys Leu Asn Asp Thr
           195                 200                 205
   Leu Leu Met Cys Leu Lys Ile Thr Ser Gly Gly Val Ile Phe Gln Ser
       210                 215                 220
   Pro Leu Met Ser Val Gln Pro Ile Asn Met Val Lys Pro Asp Pro Pro
   225                 230                 235                 240
   Leu Gly Leu His Met Glu Ile Thr Asp Asp Gly Asn Leu Lys Ile Ser
                   245                 250                 255
   Trp Ser Ser Pro Pro Leu Val Pro Phe Pro Leu Gln Tyr Gln Val Lys
               260                 265                 270
   Tyr Ser Glu Asn Ser Thr Thr Val Ile Arg Glu Ala Asp Lys Ile Val
           275                 280                 285
   Ser Ala Thr Ser Leu Leu Val Asp Ser Ile Leu Pro Gly Ser Ser Tyr
       290                 295                 300
   Glu Val Gln Val Arg Gly Lys Arg Leu Asp Gly Pro Gly Ile Trp Ser
   305                 310                 315                 320
   Asp Trp Ser Thr Pro Arg Val Phe Thr Thr Gln Asp Val Ile Tyr Phe
                   325                 330                 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465 470 475 480
   Pro Ile Ser Glu Pro Lys Asp Cys Tyr Leu Gln Ser Asp Gly Phe Tyr
   485 490 495
   Glu Cys Ile Phe Gln Pro Ile Phe Leu Leu Ser Gly Tyr Thr Met Trp
   500 505 510
   Ile Arg Ile Asn His Ser Leu Gly Ser Leu Asp Ser Pro Pro Thr Cys
   515 520 525
   Val Leu Pro Asp Ser Val Val Lys Pro Leu Pro Pro Ser Ser Val Lys
   530 535 540
   Ala Glu Ile Thr Ile Asn Ile Gly Leu Leu Lys Ile Ser Trp Glu Lys
   545 550 555 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625 630 635 640
   Arg Gly Pro Glu Phe Trp Arg Ile Ile Asn Gly Asp Thr Met Lys Lys
   645 650 655
   Glu Lys Asn Val Thr Leu Leu Trp Lys Pro Leu Met Lys Asn Asp Ser
   660 665 670
   Leu Cys Ser Val Gln Arg Tyr Val Ile Asn His His Thr Ser Cys Asn
   675 680 685
   Gly Thr Trp Ser Glu Asp Val Gly Asn His Thr Lys Phe Thr Phe Leu
   690 695 700
   Trp Thr Glu Gln Ala His Thr Val Thr Val Leu Ala Ile Asn Ser Ile
   705 710 715 720
   Gly Ala Ser Val Ala Asn Phe Asn Leu Thr Phe Ser Trp Pro Met Ser
   725 730 735
   Lys Val Asn Ile Val Gln Ser Leu Ser Ala Tyr Pro Leu Asn Ser Ser
   740 745 750
   Cys Val Ile Val Ser Trp Ile Leu Ser Pro Ser Asp Tyr Lys Leu Met
   755 760 765
   Tyr Phe Ile Ile Glu Trp Lys Asn Leu Asn Glu Asp Gly Glu Ile Lys
   770 775 780
   Trp Leu Arg Ile Ser Ser Ser Val Lys Lys Tyr Tyr Ile His Asp His
   785 790 795 800
   Phe Ile Pro Ile Glu Lys Tyr Gln Phe Ser Leu Tyr Pro Ile Phe Met
   805 810 815
   Glu Gly Val Gly Lys Pro Lys Ile Ile Asn Ser Phe Thr Gln Asp Asp
   820 825 830
   Ile Glu Lys His Gln Ser Asp Ala Gly Leu Tyr Val Ile Val Pro Val
   835 840 845
   Ile Ile Ser Ser Ser Ile Leu Leu Leu Gly Thr Leu Leu Ile Ser His
   850 855 860
   Gln Arg Met Lys Lys Leu Phe Trp Glu Asp Val Pro Asn Pro Lys Asn
   865 870 875 880
   Cys Ser Trp Ala Gln Gly Leu Asn Phe Gln Lys Pro Glu Thr Phe Glu
   885 890 895
   His Leu Phe Ile Lys His Thr Ala Ser Val Thr Cys Gly Pro Leu Leu
   900 905 910
   Leu Glu Pro Glu Thr Ile Ser Glu Asp Ile Ser Val Asp Thr Ser Trp
   915 920 925
   Lys Asn Lys Asp Glu Met Met Pro Thr Thr Val Val Ser Leu Leu Ser
   930 935 940
   Thr Thr Asp Leu Glu Lys Gly Ser Val Cys Ile Ser Asp Gln Phe Asn
   945 950 955 960
   Ser Val Asn Phe Ser Glu Ala Glu Gly Thr Glu Val Thr Tyr Glu Asp
   965 970 975
   Glu Ser Gln Arg Gln Pro Phe Val Lys Tyr Ala Thr Leu Ile Ser Asn
   980 985 990
   Ser Lys Pro Ser Glu Thr Gly Glu Glu Gln Gly Leu Ile Asn Ser Ser
   995 1000 1005
   Val Thr Lys Cys Phe Ser Ser Lys Asn Ser Pro Leu Lys Asp Ser Phe
   1010 1015 1020
   Ser Asn Ser Ser Trp Glu Ile Glu Ala Gln Ala Phe Phe Ile Leu Ser
   1025 1030 1035 1040
   Asp Gln His Pro Asn Ile Ile Ser Pro His Leu Thr Phe Ser Glu Gly
   1045 1050 1055
   Leu Asp Glu Leu Leu Lys Leu Glu Gly Asn Phe Pro Glu Glu Asn Asn
   1060 1065 1070
   Asp Lys Lys Ser Ile Tyr Tyr Leu Gly Val Thr Ser Ile Lys Lys Arg
   1075 1080 1085
   Glu Ser Gly Val Leu Leu Thr Asp Lys Ser Arg Val Ser Cys Pro Phe
   1090 1095 1100
   Pro Ala Pro Cys Leu Phe Thr Asp Ile Arg Val Leu Gln Asp Ser Cys
   1105 1110 1115 1120
   Ser His Phe Val Glu Asn Asn Ile Asn Leu Gly Thr Ser Ser Lys Lys
   1125 1130 1135
   Thr Phe Ala Ser Tyr Met Pro Gln Phe Gln Thr Cys Ser Thr Gln Thr
   1140 1145 1150
   His Lys Ile Met Glu Asn Lys Met Cys Asp Leu Thr Val Phe His Arg
   1155 1160 1165
   Asn Leu Gln Ile Cys Val Ile Met Gly Asn Ile Lys Cys Asn Arg Leu
   1170 1175 1180
   Leu Trp Val Gly Glu Arg Lys Glu Thr Arg Val Lys Phe Glu Asn Asn
   1185                1190                1195                1200
   Cys Ser Lys Lys Lys Lys Lys Lys Asn Ser Arg Pro Ala Arg Pro Asp
                   1205                1210                1215
(2) INFORMATION FOR SEQ ID NO:8:
   (i) SEQUENCE CHARACTERISTICS:
      (A) LENGTH: 3599 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:8:
   GCGGCCGCCA GTGTGATGGA TATCTGCAGA ATTCGGCTTT CTCTGCCTTC GGTCGAGTTG      60
   GACCCCCGGA TCAAGGTGTA CTTCTCTGAA GTAAGATGAT TTGTCAAAAA TTCTGTGTGG     120
   TTTTGTTACA TTGGGAATTT ATTTATGTGA TAACTGCGTT TAACTTGTCA TATCCAATTA     180
   CTCCTTGGAG ATTTAAGTTG TCTTGCATGC CACCAAATTC AACCTATGAC TACTTCCTTT     240
   TGCCTGCTGG GCTCTCAAAG AATACTTCAA ATTCGAATGG ACATTATGAG ACAGCTGTTG     300
   AACCTAAGTT TAATTCAAGT GGTACTCACT TTTCTAACTT ATCCAAAACA ACTTTCCACT     360
   GTTGCTTTCG GAGTGAGCAA GATAGAAACT GCTCCTTATG TGCAGACAAC ATTGAAGGAA     420
   AGACATTTGT TTCAACAGTA AATTCTTTAG TTTTTCAACA AATAGATGCA AACTGGAACA     480
   TACAGTGCTG GCTAAAAGGA GACTTAAAAT TATTCATCTG TTATGTGGAG TCATTATTTA     540
   AGAATCTATT CAGGAATTAT AACTATAAGG TCCATCTTTT ATATGTTCTG CCTGAAGTGT     600
   TAGAAGATTC ACCTCTGGTT CCCCAAAAAG GCAGTTTTCA GATGGTTCAC TGCAATTGCA     660
   GTGTTCACGA ATGTTGTGAA TGTCTTGTGC CTGTGCCAAC AGCCAAACTC AACGACACTC     720
   TCCTTATGTG TTTGAAAATC ACATCTGGTG GAGTAATTTT CCAGTCACCT CTAATGTCAG     780
   TTCAGCCCAT AAATATGGTG AAGCCTGATC CACCATTAGG TTTGCATATG GAAATCACAG     840
   ATGATGGTAA TTTAAAGATT TCTTGGTCCA GCCCACCATT GGTACCATTT CCACTTCAAT     900
   ATCAAGTGAA ATATTCAGAG AATTCTACAA CAGTTATCAG AGAAGCTGAC AAGATTGTCT 960
   CAGCTACATC CCTGCTAGTA GACAGTATAC TTCCTGGGTC TTCGTATGAG GTTCAGGTGA 1020
   GGGGCAAGAG ACTGGATGGC CCAGGAATCT GGAGTGACTG GAGTACTCCT CGTGTCTTTA 1080
   CCACACAAGA TGTCATATAC TTTCCACCTA AAATTCTGAC AAGTGTTGGG TCTAATGTTT 1140
   CTTTTCACTG CATCTATAAG AAGGAAAACA AGATTGTTCC CTCAAAAGAG ATTGTTTGGT 1200
   GGATGAATTT AGCTGAGAAA ATTCCTCAAA GCCAGTATGA TGTTGTGAGT GATCATGTTA 1260
   GCAAAGTTAC TTTTTTCAAT CTGAATGAAA CCAAACCTCG AGGAAAGTTT ACCTATGATG 1320
   CAGTGTACTG CTGCAATGAA CATGAATGCC ATCATCGCTA TGCTGAATTA TATGTGATTG 1380
   ATGTCAATAT CAATATCTCA TGTGAAACTG ATGGGTACTT AACTAAAATG ACTTGCAGAT 1440
   GGTCAACCAG TACAATCCAG TCACTTGCGG AAAGCACTTT GCAATTGAGG TATCATAGGA 1500
   GCAGCCTTTA CTGTTCTGAT ATTCCATCTA TTCATCCCAT ATCTGAGCCC AAAGATTGCT 1560
   ATTTGCAGAG TGATGGTTTT TATGAATGCA TTTTCCAGCC AATCTTCCTA TTATCTGGCT 1620
   ACACAATGTG GATTAGGATC AATCACTCTC TAGGTTCACT TGACTCTCCA CCAACATGTG 1680
   TCCTTCCTGA TTCTGTGGTG AAGCCACTGC CTCCATCCAG TGTGAAAGCA GAAATTACTA 1740
   TAAACATTGG ATTATTGAAA ATATCTTGGG AAAAGCCAGT CTTTCCAGAG AATAACCTTC 1800
   AATTCCAGAT TCGCTATGGT TTAAGTGGAA AAGAAGTACA ATGGAAGATG TATGAGGTTT 1860
   ATGATGCAAA ATCAAAATCT GTCAGTCTCC CAGTTCCAGA CTTGTGTGCA GTCTATGCTG 1920
   TTCAGGTGCG CTGTAAGAGG CTAGATGGAC TGGGATATTG GAGTAATTGG AGCAATCCAG 1980
   CCTACACAGT TGTCATGGAT ATAAAAGTTC CTATGAGAGG ACCTGAATTT TGGAGAATAA 2040
   TTAATGGAGA TACTATGAAA AAGGAGAAAA ATGTCACTTT ACTTTGGAAG CCCCTGATGA 2100
   AAAATGACTC ATTGTGCAGT GTTCAGAGAT ATGTGATAAA CCATCATACT TCCTGCAATG 2160
   GAACATGGTC AGAAGATGTG GGAAATCACA CGAAATTCAC TTTCCTGTGG ACAGAGCAAG 2220
   CACATACTGT TACGGTTCTG GCCATCAATT CAATTGGTGC TTCTGTTGCA AATTTTAATT 2280
   TAACCTTTTC ATGGCCTATG AGCAAAGTAA ATATCGTGCA GTCACTCAGT GCTTATCCTT 2340
   TAAACAGCAG TTGTGTGATT GTTTCCTGGA TACTATCACC CAGTGATTAC AAGCTAATGT 2400
   ATTTTATTAT TGAGTGGAAA AATCTTAATG AAGATGGTGA AATAAAATGG CTTAGAATCT 2460
   CTTCATCTGT TAAGAAGTAT TATATCCATG ATCATTTTAT CCCCATTGAG AAGTACCAGT 2520
   TCAGTCTTTA CCCAATATTT ATGGAAGGAG TGGGAAAACC AAAGATAATT AATAGTTTCA    2580
   CTCAAGATGA TATTGAAAAA CACCAGAGTG ATGCAGGTTT ATATGTAATT GTGCCAGTAA    2640
   TTATTTCCTC TTCCATCTTA TTGCTTGGAA CATTATTAAT ATCACACCAA AGAATGAAAA    2700
   AGCTATTTTG GGAAGATGTT CCGAACCCCA AGAATTGTTC CTGGGCACAA GGACTTAATT    2760
   TTCAGAAGCC AGAAACGTTT GAGCATCTTT TTATCAAGCA TACAGCATCA GTGACATGTG    2820
   GTCCTCTTCT TTTGGAGCCT GAAACAATTT CAGAAGATAT CAGTGTTGAT ACATCATGGA    2880
   AAAATAAAGA TGAGATGATG CCAACAACTG TGGTCTCTCT ACTTTCAACA ACAGATCTTG    2940
   AAAAGGGTTC TGTTTGTATT AGTGACCAGT TCAACAGTGT TAACTTCTCT GAGGCTGAGG    3000
   GTACTGAGGT AACCTATGAG GACGAAAGCC AGAGACAACC CTTTGTTAAA TACGCCACGC    3060
   TGATCAGCAA CTCTAAACCA AGTGAAACTG GTGAAGAACA AGGGCTTATA AATAGTTCAG    3120
   TCACCAAGTG CTTCTCTAGC AAAAATTCTC CGTTGAAGGA TTCTTTCTCT AATAGCTCAT    3180
   GGGAGATAGA GGCCCAGGCA TTTTTTATAT TATCGGATCA GCATCCCAAC ATAATTTCAC    3240
   CACACCTCAC ATTCTCAGAA GGATTGGATG AACTTTTGAA ATTGGAGGGA AATTTCCCTG    3300
   AAGAAAATAA TGATAAAAAG TCTATCTATT ATTTAGGGGT CACCTCAATC AAAAAGAGAG    3360
   AGAGTGGTGT GCTTTTGACT GACAAGTCAA GGGTATCGTG CCCATTCCCA GCCCCCTGTT    3420
   TATTCACGGA CATCAGAGTT CTCCAGGACA GTTGCTCACA CTTTGTAGAA AATAATATCA    3480
   ACTTAGGAAC TTCTAGTAAG AAGACTTTTG CATCTTACAT GCCTCAATTC CAAACTTGTT    3540
   CTACTCAGAC TCATAAGATC ATGGAAAACA AGATGTGTGA CCTAACTGTG TAATCTAGA     3599
(2) INFORMATION FOR SEQ ID NO:9:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
   NNNNNTACCT TTTCCAG                                                   17
(2) INFORMATION FOR SEQ ID NO:10:
   (i) SEQUENCE CHARACTERISTICS:
      (A) LENGTH: 839 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:10:
   Met Ile Cys Gln Lys Phe Cys Val Val Leu Leu His Trp Glu Phe Ile
   1               5                   10                  15
   Tyr Val Ile Thr Ala Phe Asn Leu Ser Tyr Pro Ile Thr Pro Trp Arg
               20                  25                  30
   Phe Lys Leu Ser Cys Met Pro Pro Asn Ser Thr Tyr Asp Tyr Phe Leu
           35                  40                  45
   Leu Pro Ala Gly Leu Ser Lys Asn Thr Ser Asn Ser Asn Gly His Tyr
       50                  55                  60
   Glu Thr Ala Val Glu Pro Lys Phe Asn Ser Ser Gly Thr His Phe Ser
   65                  70                  75                  80
   Asn Leu Ser Lys Thr Thr Phe His Cys Cys Phe Arg Ser Glu Gln Asp
                   85                  90                  95
   Arg Asn Cys Ser Leu Cys Ala Asp Asn Ile Glu Gly Lys Thr Phe Val
               100                 105                 110
   Ser Thr Val Asn Ser Leu Val Phe Gln Gln Ile Asp Ala Asn Trp Asn
           115                 120                 125
   Ile Gln Cys Trp Leu Lys Gly Asp Leu Lys Leu Phe Ile Cys Tyr Val
       130                 135                 140
   Glu Ser Leu Phe Lys Asn Leu Phe Arg Asn Tyr Asn Tyr Lys Val His
   145                 150                 155                 160
   Leu Leu Tyr Val Leu Pro Glu Val Leu Glu Asp Ser Pro Leu Val Pro
                   165                 170                 175
   Gln Lys Gly Ser Phe Gln Met Val His Cys Asn Cys Ser Val His Glu
   180 185 190
   Cys Cys Glu Cys Leu Val Pro Val Pro Thr Ala Lys Leu Asn Asp Thr
   195 200 205
   Leu Leu Met Cys Leu Lys Ile Thr Ser Gly Gly Val Ile Phe Gln Ser
   210 215 220
   Pro Leu Met Ser Val Gln Pro Ile Asn Met Val Lys Pro Asp Pro Pro
   225 230 235 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
   275 280 285
   Ser Ala Thr Ser Leu Leu Val Asp Ser Ile Leu Pro Gly Ser Ser Tyr
   290 295 300
   Glu Val Gln Val Arg Gly Lys Arg Leu Asp Gly Pro Gly Ile Trp Ser
   305 310 315 320
   Asp Trp Ser Thr Pro Arg Val Phe Thr Thr Gln Asp Val Ile Tyr Phe
   325 330 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465                 470                 475                 480
   Pro Ile Ser Glu Pro Lys Asp Cys Tyr Leu Gln Ser Asp Gly Phe Tyr
                   485                 490                 495
   Glu Cys Ile Phe Gln Pro Ile Phe Leu Leu Ser Gly Tyr Thr Met Trp
               500                 505                 510
   Ile Arg Ile Asn His Ser Leu Gly Ser Leu Asp Ser Pro Pro Thr Cys
           515                 520                 525
   Val Leu Pro Asp Ser Val Val Lys Pro Leu Pro Pro Ser Ser Val Lys
       530                 535                 540
   Ala Glu Ile Thr Ile Asn Ile Gly Leu Leu Lys Ile Ser Trp Glu Lys
   545                 550                 555                 560
   Pro Val Phe Pro Glu Asn Asn Leu Gln Phe Gln Ile Arg Tyr Gly Leu
                   565                 570                 575
   Ser Gly Lys Glu Val Gln Trp Lys Met Tyr Glu Val Tyr Asp Ala Lys
               580                 585                 590
   Ser Lys Ser Val Ser Leu Pro Val Pro Asp Leu Cys Ala Val Tyr Ala
           595                 600                 605
   Val Gln Val Arg Cys Lys Arg Leu Asp Gly Leu Gly Tyr Trp Ser Asn
       610                 615                 620
   Trp Ser Asn Pro Ala Tyr Thr Val Val Met Asp Ile Lys Val Pro Met
   625                 630                 635                 640
   Arg Gly Pro Glu Phe Trp Arg Ile Ile Asn Gly Asp Thr Met Lys Lys
                   645                 650                 655
   Glu Lys Asn Val Thr Leu Leu Trp Lys Pro Leu Met Lys Asn Asp Ser
               660                 665                 670
   Leu Cys Ser Val Gln Arg Tyr Val Ile Asn His His Thr Ser Cys Asn
           675                 680                 685
   Gly Thr Trp Ser Glu Asp Val Gly Asn His Thr Lys Phe Thr Phe Leu
       690                 695                 700
   Trp Thr Glu Gln Ala His Thr Val Thr Val Leu Ala Ile Asn Ser Ile
   705                 710                 715                 720
   Gly Ala Ser Val Ala Asn Phe Asn Leu Thr Phe Ser Trp Pro Met Ser
                   725                 730                 735
   Lys Val Asn Ile Val Gln Ser Leu Ser Ala Tyr Pro Leu Asn Ser Ser
               740                 745                 750
   Cys Val Ile Val Ser Trp Ile Leu Ser Pro Ser Asp Tyr Lys Leu Met
           755                 760                 765
   Tyr Phe Ile Ile Glu Trp Lys Asn Leu Asn Glu Asp Gly Glu Ile Lys
       770                 775                 780
   Trp Leu Arg Ile Ser Ser Ser Val Lys Lys Tyr Tyr Ile His Asp His
   785                 790                 795                 800
   Phe Ile Pro Ile Glu Lys Tyr Gln Phe Ser Leu Tyr Pro Ile Phe Met
                   805                 810                 815
   Glu Gly Val Gly Lys Pro Lys Ile Ile Asn Ser Phe Thr Gln Asp Asp
               820                 825                 830
   Ile Glu Lys His Gln Ser Asp
           835
(2) INFORMATION FOR SEQ ID NO: 11
   (i) SEQUENCE CHARACTERISTICS:
      (A) LENGTH: 2624 base pairs
      (B) TYPE: nucleid acid
      (C) STRANDEDNESS: single
      (D) TOPOLOGY: linear
   (ii) MOLECULE TYPE: cDNA
   (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 2948 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
   CCATTGAAGT CAATGGGAGT TTGTTTTGGC ACCAAAATCA ACGGGGATTT CCAAAATGTC     60
   GTAATAACCC CGCCCCGTTG ACGCAAATGG GCGGTAGGCG TGTACGGTGG GAGGTCTATA    120
   TAAGCAGAGC TCGTTTAGTG AACCGTCAGA TCTCTAGAAG CTGGGTACCA GCTGCTAGCA    180
   AGCTTGCTAG CGGCCGCCAG TGTGATGGAT ATCTGCAGAA TTCGGCTTTC TCTGCCTTCG    240
   GTCGAGTTGG ACCCCCGGAT CAAGGTGTAC TTCTCTGAAG TAAGATGATT TGTCAAAAAT    300
   TCTGTGTGGT TTTGTTACAT TGGGAATTTA TTTATGTGAT AACTGCGTTT AACTTGTCAT    360
   ATCCAATTAC TCCTTGGAGA TTTAAGTTGT CTTGCATGCC ACCAAATTCA ACCTATGACT    420
   ACTTCCTTTT GCCTGCTGGG CTCTCAAAGA ATACTTCAAA TTCGAATGGA CATTATGAGA    480
   CAGCTGTTGA ACCTAAGTTT AATTCAAGTG GTACTCACTT TTCTAACTTA TCCAAAACAA    540
   CTTTCCACTG TTGCTTTCGG AGTGAGCAAG ATAGAAACTG CTCCTTATGT GCAGACAACA    600
   TTGAAGGAAA GACATTTGTT TCAACAGTAA ATTCTTTAGT TTTTCAACAA ATAGATGCAA   660
   ACTGGAACAT ACAGTGCTGG CTAAAAGGAG ACTTAAAATT ATTCATCTGT TATGTGGAGT   720
   CATTATTTAA GAATCTATTC AGGAATTATA ACTATAAGGT CCATCTTTTA TATGTTCTGC   780
   CTGAAGTGTT AGAAGATTCA CCTCTGGTTC CCCAAAAAGG CAGTTTTCAG ATGGTTCACT   840
   GCAATTGCAG TGTTCACGAA TGTTGTGAAT GTCTTGTGCC TGTGCCAACA GCCAAACTCA   900
   ACGACACTCT CCTTATGTGT TTGAAAATCA CATCTGGTGG AGTAATTTTC CAGTCACCTC   960
   TAATGTCAGT TCAGCCCATA AATATGGTGA AGCCTGATCC ACCATTAGGT TTGCATATGG  1020
   AAATCACAGA TGATGGTAAT TTAAAGATTT CTTGGTCCAG CCCACCATTG GTACCATTTC  1080
   CACTTCAATA TCAAGTGAAA TATTCAGAGA ATTCTACAAC AGTTATCAGA GAAGCTGACA  1140
   AGATTGTCTC AGCTACATCC CTGCTAGTAG ACAGTATACT TCCTGGGTCT TCGTATGAGG  1200
   TTCAGGTGAG GGGCAAGAGA CTGGATGGCC CAGGAATCTG GAGTGACTGG AGTACTCCTC  1260
   GTGTCTTTAC CACACAAGAT GTCATATACT TTCCACCTAA AATTCTGACA AGTGTTGGGT  1320
   CTAATGTTTC TTTTCACTGC ATCTATAAGA AGGAAAACAA GATTGTTCCC TCAAAAGAGA  1380
   TTGTTTGGTG GATGAATTTA GCTGAGAAAA TTCCTCAAAG CCAGTATGAT GTTGTGAGTG  1440
   ATCATGTTAG CAAAGTTACT TTTTTCAATC TGAATGAAAC CAAACCTCGA GGAAAGTTTA  1500
   CCTATGATGC AGTGTACTGC TGCAATGAAC ATGAATGCCA TCATCGCTAT GCTGAATTAT  1560
   ATGTGATTGA TGTCAATATC AATATCTCAT GTGAAACTGA TGGGTACTTA ACTAAAATGA  1620
   CTTGCAGATG GTCAACCAGT ACAATCCAGT CACTTGCGGA AAGCACTTTG CAATTGAGGT  1680
   ATCATAGGAG CAGCCTTTAC TGTTCTGATA TTCCATCTAT TCATCCCATA TCTGAGCCCA  1740
   AAGATTGCTA TTTGCAGAGT GATGGTTTTT ATGAATGCAT TTTCCAGCCA ATCTTCCTAT  1800
   TATCTGGCTA CACAATGTGG ATTAGGATCA ATCACTCTCT AGGTTCACTT GACTCTCCAC  1860
   CAACATGTGT CCTTCCTGAT TCTGTGGTGA AGCCACTGCC TCCATCCAGT GTGAAAGCAG  1920
   AAATTACTAT AAACATTGGA TTATTGAAAA TATCTTGGGA AAAGCCAGTC TTTCCAGAGA  1980
   ATAACCTTCA ATTCCAGATT CGCTATGGTT TAAGTGGAAA AGAAGTACAA TGGAAGATGT  2040
   ATGAGGTTTA TGATGCAAAA TCAAAATCTG TCAGTCTCCC AGTTCCAGAC TTGTGTGCAG  2100
   TCTATGCTGT TCAGGTGCGC TGTAAGAGGC TAGATGGACT GGGATATTGG AGTAATTGGA  2160
   GCAATCCAGC CTACACAGTT GTCATGGATA TAAAAGTTCC TATGAGAGGA CCTGAATTTT  2220
   GGAGAATAAT TAATGGAGAT ACTATGAAAA AGGAGAAAAA TGTCACTTTA CTTTGGAAGC    2280
   CCCTGATGAA AAATGACTCA TTGTGCAGTG TTCAGAGATA TGTGATAAAC CATCATACTT    2340
   CCTGCAATGG AACATGGTCA GAAGATGTGG GAAATCACAC GAAATTCACT TTCCTGTGGA    2400
   CAGAGCAAGC ACATACTGTT ACGGTTCTGG CCATCAATTC AATTGGTGCT TCTGTTGCAA    2460
   ATTTTAATTT AACCTTTTCA TGGCCTATGA GCAAAGTAAA TATCGTGCAG TCACTCAGTG    2520
   CTTATCCTTT AAACAGCAGT TGTGTGATTG TTTCCTGGAT ACTATCACCC AGTGATTACA    2580
   AGCTAATGTA TTTTATTATT GAGTGGAAAA ATCTTAATGA AGATGGTGAA ATAAAATGGC    2640
   TTAGAATCTC TTCATCTGTT AAGAAGTATT ATATCCATGA TCATTTTATC CCCATTGAGA    2700
   AGTACCAGTT CAGTCTTTAC CCAATATTTA TGGAAGGAGT GGGAAAACCA AAGATAATTA    2760
   ATAGTTTCAC TCAAGATGAT ATTGAAAAAC ACCAGAGTGA TGCAGGTGAC TACAAGGACG    2820
   ACGATGACAA GTAGGGATCC AGACATGATA AGATACATTG ATGAGTTTGG ACAACCCACA    2880
   ACTAGAATGC AGTGAAAAAA ATGCTTTATT TGTGAAATTT GTGATGCTAT TGCTTTATTT    2940
   GTAACCAT                                                             2948
(2) INFORMATION FOR SEQ ID NO:13:
   (i) SEQUENCE CHARACTERISTICS:
      (A) LENGTH: 804 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:13:
   Met Ile Cys Gln Lys Phe Cys Val Val Leu Leu His Trp Glu Phe Ile
   1               5                   10                  15
   Tyr Val Ile Thr Ala Phe Asn Leu Ser Tyr Pro Ile Thr Pro Trp Arg
               20                  25                  30
   Phe Lys Leu Ser Cys Met Pro Pro Asn Ser Thr Tyr Asp Tyr Phe Leu
           35                  40                  45
   Leu Pro Ala Gly Leu Ser Lys Asn Thr Ser Asn Ser Asn Gly His Tyr
       50                  55                  60
   Glu Thr Ala Val Glu Pro Lys Phe Asn Ser Ser Gly Thr His Phe Ser
   65 70 75 80
   Asn Leu Ser Lys Thr Thr Phe His Cys Cys Phe Arg Ser Glu Gln Asp
   85 90 95
   Arg Asn Cys Ser Leu Cys Ala Asp Asn Ile Glu Gly Lys Thr Phe Val
   100 105 110
   Ser Thr Val Asn Ser Leu Val Phe Gln Gln Ile Asp Ala Asn Trp Asn
   115 120 125
   Ile Gln Cys Trp Leu Lys Gly Asp Leu Lys Leu Phe Ile Cys Tyr Val
   130 135 140
   Glu Ser Leu Phe Lys Asn Leu Phe Arg Asn Tyr Asn Tyr Lys Val His
   145 150 155 160
   Leu Leu Tyr Val Leu Pro Glu Val Leu Glu Asp Ser Pro Leu Val Pro
   165 170 175
   Gln Lys Gly Ser Phe Gln Met Val His Cys Asn Cys Ser Val His Glu
   180 185 190
   Cys Cys Glu Cys Leu Val Pro Val Pro Thr Ala Lys Leu Asn Asp Thr
   195 200 205
   Leu Leu Met Cys Leu Lys Ile Thr Ser Gly Gly Val Ile Phe Gln Ser
   210 215 220
   Pro Leu Met Ser Val Gln Pro Ile Asn Met Val Lys Pro Asp Pro Pro
   225 230 235 240
   Leu Gly Leu His Met Glu Ile Thr Asp Asp Gly Asn Leu Lys Ile Ser
   245 250 255
   Trp Ser Ser Pro Pro Leu Val Pro Phe Pro Leu Gln Tyr Gln Val Lys
   260 265 270
   Tyr Ser Glu Asn Ser Thr Thr Val Ile Arg Glu Ala Asp Lys Ile Val
   275 280 285
   Ser Ala Thr Ser Leu Leu Val Asp Ser Ile Leu Pro Gly Ser Ser Tyr
   290 295 300
   Glu Val Gln Val Arg Gly Lys Arg Leu Asp Gly Pro Gly Ile Trp Ser
   305 310 315 320
   Asp Trp Ser Thr Pro Arg Val Phe Thr Thr Gln Asp Val Ile Tyr Phe
   325 330 335
   Pro Pro Lys Ile Leu Thr Ser Val Gly Ser Asn Val Ser Phe His Cys
   340 345 350
   Ile Tyr Lys Lys Glu Asn Lys Ile Val Pro Ser Lys Glu Ile Val Trp
   355 360 365
   Trp Met Asn Leu Ala Glu Lys Ile Pro Gln Ser Gln Tyr Asp Val Val
   370 375 380
   Ser Asp His Val Ser Lys Val Thr Phe Phe Asn Leu Asn Glu Thr Lys
   385 390 395 400
   Pro Arg Gly Lys Phe Thr Tyr Asp Ala Val Tyr Cys Cys Asn Glu His
   405 410 415
   Glu Cys His His Arg Tyr Ala Glu Leu Tyr Val Ile Asp Val Asn Ile
   420 425 430
   Asn Ile Ser Cys Glu Thr Asp Gly Tyr Leu Thr Lys Met Thr Cys Arg
   435 440 445
   Trp Ser Thr Ser Thr Ile Gln Ser Leu Ala Glu Ser Thr Leu Gln Leu
   450 455 460
   Arg Tyr His Arg Ser Ser Leu Tyr Cys Ser Asp Ile Pro Ser Ile His
   465 470 475 480
   Pro Ile Ser Glu Pro Lys Asp Cys Tyr Leu Gln Ser Asp Gly Phe Tyr
   485 490 495
   Glu Cys Ile Phe Gln Pro Ile Phe Leu Leu Ser Gly Tyr Thr Met Trp
   500 505 510
   Ile Arg Ile Asn His Ser Leu Gly Ser Leu Asp Ser Pro Pro Thr Cys
   515 520 525
   Val Leu Pro Asp Ser Val Val Lys Pro Leu Pro Pro Ser Ser Val Lys
   530 535 540
   Ala Glu Ile Thr Ile Asn Ile Gly Leu Leu Lys Ile Ser Trp Glu Lys
   545 550 555 560
   Pro Val Phe Pro Glu Asn Asn Leu Gln Phe Gln Ile Arg Tyr Gly Leu
   565 570 575
   Ser Gly Lys Glu Val Gln Trp Lys Met Tyr Glu Val Tyr Asp Ala Lys
   580 585 590
   Ser Lys Ser Val Ser Leu Pro Val Pro Asp Leu Cys Ala Val Tyr Ala
   595 600 605
   Val Gln Val Arg Cys Lys Arg Leu Asp Gly Leu Gly Tyr Trp Ser Asn
   610 615 620
   Trp Ser Asn Pro Ala Tyr Thr Val Val Met Asp Ile Lys Val Pro Met
   625                 630                 635                 640
   Arg Gly Pro Glu Phe Trp Arg Ile Ile Asn Gly Asp Thr Met Lys Lys
                   645                 650                 655
   Glu Lys Asn Val Thr Leu Leu Trp Lys Pro Leu Met Lys Asn Asp Ser
               660                 665                 670
   Leu Cys Ser Val Gln Arg Tyr Val Ile Asn His His Thr Ser Cys Asn
           675                 680                 685
   Gly Thr Trp Ser Glu Asp Val Gly Asn His Thr Lys Phe Thr Phe Leu
       690                 695                 700
   Trp Thr Glu Gln Ala His Thr Val Thr Val Leu Ala Ile Asn Ser Ile
   705                 710                 715                 720
   Gly Ala Ser Val Ala Asn Phe Asn Leu Thr Phe Ser Trp Pro Met Ser
                   725                 730                 735
   Lys Val Asn Ile Val Gln Ser Leu Ser Ala Tyr Pro Leu Asn Ser Ser
               740                 745                 750
   Cys Val Ile Val Ser Trp Ile Leu Ser Pro Ser Asp Tyr Lys Leu Met
           755                 760                 765
   Tyr Phe Ile Ile Glu Trp Lys Asn Leu Asn Glu Asp Gly Glu Ile Lys
       770                 775                 780
   Trp Leu Arg Ile Ser Ser Ser Val Lys Lys Tyr Tyr Ile His Gly Lys
   785                 790                 795                 800
   Phe Thr Ile Leu
(2) INFORMATION FOR SEQ ID NO:14:
   (i) SEQUENCE CHARACTERISTICS:
      (A) LENGTH: 2507 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   GCGGCCGCCA GTGTGATGGA TATCTGCAGA ATTCGGCTTT CTCTGCCTTC GGTCGAGTTG      60
   GACCCCCGGA TCAAGGTGTA CTTCTCTGAA GTAAGATGAT TTGTCAAAAA TTCTGTGTGG     120
   TTTTGTTACA TTGGGAATTT ATTTATGTGA TAACTGCGTT TAACTTGTCA TATCCAATTA     180
   CTCCTTGGAG ATTTAAGTTG TCTTGCATGC CACCAAATTC AACCTATGAC TACTTCCTTT     240
   TGCCTGCTGG GCTCTCAAAG AATACTTCAA ATTCGAATGG ACATTATGAG ACAGCTGTTG     300
   AACCTAAGTT TAATTCAAGT GGTACTCACT TTTCTAACTT ATCCAAAACA ACTTTCCACT     360
   GTTGCTTTCG GAGTGAGCAA GATAGAAACT GCTCCTTATG TGCAGACAAC ATTGAAGGAA     420
   AGACATTTGT TTCAACAGTA AATTCTTTAG TTTTTCAACA AATAGATGCA AACTGGAACA     480
   TACAGTGCTG GCTAAAAGGA GACTTAAAAT TATTCATCTG TTATGTGGAG TCATTATTTA     540
   AGAATCTATT CAGGAATTAT AACTATAAGG TCCATCTTTT ATATGTTCTG CCTGAAGTGT     600
   TAGAAGATTC ACCTCTGGTT CCCCAAAAAG GCAGTTTTCA GATGGTTCAC TGCAATTGCA     660
   GTGTTCACGA ATGTTGTGAA TGTCTTGTGC CTGTGCCAAC AGCCAAACTC AACGACACTC     720
   TCCTTATGTG TTTGAAAATC ACATCTGGTG GAGTAATTTT CCAGTCACCT CTAATGTCAG     780
   TTCAGCCCAT AAATATGGTG AAGCCTGATC CACCATTAGG TTTGCATATG GAAATCACAG     840
   ATGATGGTAA TTTAAAGATT TCTTGGTCCA GCCCACCATT GGTACCATTT CCACTTCAAT     900
   ATCAAGTGAA ATATTCAGAG AATTCTACAA CAGTTATCAG AGAAGCTGAC AAGATTGTCT     960
   CAGCTACATC CCTGCTAGTA GACAGTATAC TTCCTGGGTC TTCGTATGAG GTTCAGGTGA    1020
   GGGGCAAGAG ACTGGATGGC CCAGGAATCT GGAGTGACTG GAGTACTCCT CGTGTCTTTA    1080
   CCACACAAGA TGTCATATAC TTTCCACCTA AAATTCTGAC AAGTGTTGGG TCTAATGTTT    1140
   CTTTTCACTG CATCTATAAG AAGGAAAACA AGATTGTTCC CTCAAAAGAG ATTGTTTGGT    1200
   GGATGAATTT AGCTGAGAAA ATTCCTCAAA GCCAGTATGA TGTTGTGAGT GATCATGTTA    1260
   GCAAAGTTAC TTTTTTCAAT CTGAATGAAA CCAAACCTCG AGGAAAGTTT ACCTATGATG    1320
   CAGTGTACTG CTGCAATGAA CATGAATGCC ATCATCGCTA TGCTGAATTA TATGTGATTG    1380
   ATGTCAATAT CAATATCTCA TGTGAAACTG ATGGGTACTT AACTAAAATG ACTTGCAGAT    1440
   GGTCAACCAG TACAATCCAG TCACTTGCGG AAAGCACTTT GCAATTGAGG TATCATAGGA    1500
   GCAGCCTTTA CTGTTCTGAT ATTCCATCTA TTCATCCCAT ATCTGAGCCC AAAGATTGCT    1560
   ATTTGCAGAG TGATGGTTTT TATGAATGCA TTTTCCAGCC AATCTTCCTA TTATCTGGCT    1620
   ACACAATGTG GATTAGGATC AATCACTCTC TAGGTTCACT TGACTCTCCA CCAACATGTG    1680
   TCCTTCCTGA TTCTGTGGTG AAGCCACTGC CTCCATCCAG TGTGAAAGCA GAAATTACTA    1740
   TAAACATTGG ATTATTGAAA ATATCTTGGG AAAAGCCAGT CTTTCCAGAG AATAACCTTC    1800
   AATTCCAGAT TCGCTATGGT TTAAGTGGAA AAGAAGTACA ATGGAAGATG TATGAGGTTT    1860
   ATGATGCAAA ATCAAAATCT GTCAGTCTCC CAGTTCCAGA CTTGTGTGCA GTCTATGCTG    1920
   TTCAGGTGCG CTGTAAGAGG CTAGATGGAC TGGGATATTG GAGTAATTGG AGCAATCCAG    1980
   CCTACACAGT TGTCATGGAT ATAAAAGTTC CTATGAGAGG ACCTGAATTT TGGAGAATAA    2040
   TTAATGGAGA TACTATGAAA AAGGAGAAAA ATGTCACTTT ACTTTGGAAG CCCCTGATGA    2100
   AAAATGACTC ATTGTGCAGT GTTCAGAGAT ATGTGATAAA CCATCATACT TCCTGCAATG    2160
   GAACATGGTC AGAAGATGTG GGAAATCACA CGAAATTCAC TTTCCTGTGG ACAGAGCAAG    2220
   CACATACTGT TACGGTTCTG GCCATCAATT CAATTGGTGC TTCTGTTGCA AATTTTAATT    2280
   TAACCTTTTC ATGGCCTATG AGCAAAGTAA ATATCGTGCA GTCACTCAGT GCTTATCCTT    2340
   TAAACAGCAG TTGTGTGATT GTTTCCTGGA TACTATCACC CAGTGATTAC AAGCTAATGT    2400
   ATTTTATTAT TGAGTGGAAA AATCTTAATG AAGATGGTGA AATAAAATGG CTTAGAATCT    2460
   CTTCATCTGT TAAGAAGTAT TATATCCATG GTAAGTTTAC TATACTT                  2507
(2) INFORMATION FOR SEQ ID NO:15:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   GTAAGTTATT TGNNNNNATA TCCTAACAG                                      29
(2) INFORMATION FOR SEQ ID NO:16:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   GTAAGCATTA GCNNNNNTTT TAAATTCAG 29
(2) INFORMATION FOR SEQ ID NO:17:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
   GTAAGTACCA AANNNNNTTT TCAATATAG 29
(2) INFORMATION FOR SEQ ID NO:18:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
   GTAAGTTATG CANNNNNTTT TTCCTTAAG 29
(2) INFORMATION FOR SEQ ID NO:19:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
   GTAAGTATAT TTNNNNAATA TTTAACAG 28
(2) INFORMATION FOR SEQ ID NO:20:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
   GTAGGTTATG TANNNNNCCC TCATTACAG 29
(2) INFORMATION FOR SEQ ID NO:21:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   GTAAGAAAAC AGNNNNNTGT TTCAAATAG 29
(2) INFORMATION FOR SEQ ID NO:22:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
   GTACGTATTA TTNNNNNTAT CTTTTAAAG 29
(2) INFORMATION FOR SEQ ID NO:23:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
   GTATGTCAAG CTNNNNNAAA AATTTCTAG 29
(2) INFORMATION FOR SEQ ID NO:24:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
   GTACCTTTTA CTNNNNNCTT ATTTTACAG 29
(2) INFORMATION FOR SEQ ID NO:25:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:25:
   GTCTGCAGAG ATNNNNNGTC ATTTTGCAG 29
(2) INFORMATION FOR SEQ ID NO:26:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:26:
   GTATTCCCAA TTNNNNNTAT TTACTACAG 29
(2) INFORMATION FOR SEQ ID NO:27:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:27:
   GTATTCCCAA TTNNNNNTAT TTACTACAG 29
(2) INFORMATION FOR SEQ ID NO:28:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:28:
   GTAAGTTTAC TANNNNNTTT TCTCCTCAG 29
(2) INFORMATION FOR SEQ ID NO:29:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:29:
   GTAAAAATTA TANNNNNTTT CTTTTTCAG 29
(2) INFORMATION FOR SEQ ID NO:30:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:30:
   GTATTGTACT TGNNNNNTAT CCTTTGTAG 29
(2) INFORMATION FOR SEQ ID NO:31:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:31:
   GTTGCTTTTT CANNNNNTTA TCTAAACAG 29
(2) INFORMATION FOR SEQ ID NO:32:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:32:
   GTACATTTGT CTNNNNNCTT TTCTTTTAG 29
(2) INFORMATION FOR SEQ ID NO:33:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:33:
   GTATCCAGTG TTNNNNNCTT TTTAAACAG 29

## Description

### FIELD OF THE INVENTION

The present invention relates to OB protein receptors, related compositions and methods of making and using such receptors and related compositions.

### BACKGROUND

Although the molecular basis for obesity is largely unknown, the identification of the "OB gene" and protein encoded ("OB protein") has shed some light on mechanisms the body uses to regulate body fat deposition. Zhang et al., Nature 372: 425-432 (1994); see also, the Correction at Nature 374: 479 (1995). The OB protein is active in vivo in both *ob*/*ob* mutant mice (mice obese due to a defect in the production of the OB gene product) as well as in normal, wild type mice. The biological activity manifests itself in, among other things, weight loss. See generally, Barinaga, "Obese" Protein Slims Mice, Science 269: 475-476 (1995). See PCT International Publication Number WO 96/05309, "Modulators of Body Weight, Corresponding Nucleic Acids and Proteins, and Diagnostic and Therapeutic Uses Thereof," herein incorporated by reference.

The other biological effects of OB protein are not well characterized. It is known, for instance, that in *ob*/*ob* mutant mice, administration of OB protein results in a decrease in serum insulin levels, and serum glucose levels. It is also known that administration of OB protein results in a decrease in body fat. This was observed in both ob/ob mutant mice, as well as non-obese normal mice. Pelleymounter et al., Science 269: 540-543 (1995); Halaas et al., Science 269: 543-546 (1995). See also, Campfield et al., Science 269: 546-549 (1995) (Peripheral and central administration of microgram doses of OB protein reduced food intake and body weight of *ob*/*ob* and diet-induced obese mice but not in *db*/*db* obese mice.) In none of these reports have toxicities been observed, even at the highest doses.

Despite the promise of clinical application of the OB protein, the mode of action of the OB protein in vivo is not clearly elucidated, in part due to the absence of information on the OB receptor. High affinity binding of the OB protein has been detected in the rat hypothalamus, reportedly indicating OB receptor location. Stephens et al., Nature 377: 530-532 (1995). The *db*/*db* mouse displays the identical phenotype as the *ob*/*ob* mouse, i.e., extreme obesity and Type II diabetes; this phenotype is thought to be due to a defective OB receptor, particularly since *db*/*db* mice fail to respond to OB protein administration. See Stephens et al., supra.

Identification of the OB protein receptor is key in determining the pathway of signal transduction. Moreover, identification of the OB protein receptor would provide powerful application in diagnostic uses, for example, to determine if individuals would benefit from OB protein therapy. Furthermore, the OB receptor could be a key component in an assay for determining additional molecules which bind to the receptor and result in desired biological activity. Further, such soluble receptor could enhance or alter the effectiveness of OB protein (or analog or derivative thereof).

### SUMMARY OF THE INVENTION

The present invention relates to a novel class of protein receptors, herein denominated "OB protein receptors" or "OB receptors", which are thought to selectively bind OB protein. As such, the novel OB receptor family is provided, as well as novel members of such family. Also provided are nucleic acids, vectors and host cells containing such nucleic acids, related antisense nucleic acids, molecules which selectively bind to the OB protein receptor, and related compositions of matter, such as OB receptor protein/OB protein complexes. In other aspects, the present invention relates to methods of using the above compositions, such as therapeutic and/or diagnostic methods, and methods for preparing OB receptor ligands.

### DETAILED DESCRIPTION

A novel family of OB receptors is provided. This novel family resulted from identification of a PCR fragment isolated from a human liver cell cDNA library. The original PCR fragment, from which primers were isolated, contained a "WSXWS" motif, common to cytokine receptors. As illustrated by the working examples below, using this fragment four members of this OB protein receptor family have been identified. These members, herein designated as "A", "B", and "C", and "D" are indentical at amino acid position 1-891 (using the numbering of Seq. ID No. 1), but diverge at position 892 through the C-terminus. They vary in length at the C-terminus beyond amino acid 891, and the different forms appear to have different tissue distribution.

Using hydrophobicity analysis, the leader sequence is likely to comprise amino acids (Seq. ID. No. 1) 1-21, 1-22, or 1-28. The first amino acid of the mature protein is likely to be 22 (F), 23 (N) or 29 (T). Most likely, based on analysis of eucaryotic cell expression (CHO cell expression see Example 8, infra), the first amino acid of the mature protein is 22(F). The beginning of the transmembrane domain appears to be located at position 840 (A) or 842 (L). The end of the transmembrane domain appears to be located at position 862 (I), 863 (S) or 864 (H). Thus, based on predictions from hydrophobicity analysis, for OB protein binding, at a minimum what is needed is the extracellular domain of the mature protein, amino acids 22, 23 or 29 through amino acids 839 (D) or 841 (G). Therefore, the present class of OB receptor proteins is those having amino acids (according to Seq. ID No. 1):
(a) 1-896;
(b) 22-896;
(c) 23-896;
(d) 29-896;
(e) 22-891;
(f) 23-891;
(g) 29-891;
(h) sequences (e) through (g) having the C-terminal amino acids selected from among:
   (i) OB receptor B (Seq. ID No. 3) positions 892-904;
   (ii) OB receptor C (Seq. ID No. 5) positions 892- 958; and,
(i) amino acids of subparts b, c, d, e, f, g, and (h) lacking a leader sequence, which have an N-terminal methionyl residue.
(j) a chemically modified derivative of any of subparts (a) through (h).

The functional domains of the OB receptor may be predicted using the information contained in Bazan et al., PNAS-USA 87: 6934-6938 (1990) (incorporated herein by reference). For the present OB receptor, there are two hematopoietin domains, a random coil region, the transmembrane domain, and the intracellular domain. The overall geography may be illustrated as follows:

Using the information provided by Bazan, supra, the domains may be predicted, with essentially an error of approximately plus or minus three base pairs (as applied to all amino acid location specified for purposes of identifying the Bazan predicted domains). The precise locations may be determined empirically by methods known in the art, such as preparing and expressing modified recombinant DNAs. The structural characteristics are though to be important for maintaining the structural integrity of the molecule, and therefore, to the extent that such structure is important for function, for functional characteristics as well.

The hematopoietin domains (H1 and H2) are thought to have two fibronectin type 3 repeats each, one set of paired cysteine residues each (thought to form a disulfide bridge), and one "WSXWS box" (referring to the single letter amino acid abbreviation, with "X" being any amino acid). The fibrinectin type 3 domains may be identified by location of a double proline ("PP"), which marks the beginning of the second fibronectin type 3 repeat; the actual beginning of such second fibronectin type 3 repeat is likely to begin about 3 amino acids upstream of that double proline.

The first hematopoietin domain is likely to begin at amino acid 123 (using the numbering according to Seq. ID No. 1, for example), which is an isoleucine residue (I). The last amino acid of the hematopoietin domain is likely to be amino acid 339, which is a lysine (K) residue. The two fibronectin type 3 repeats are likely to be located at (about) amino acids 123 through 235 and 236 through 339. There is a single pair of cysteine residues which likely form a disulfide bridge, located at position 131 and position 142. The "WSXWS box" is located at position 319 through 323.

The second hematopoietin domain is likely to begin at position 428, which is an isoleucine (I) and end at position 642 which is a glycine (G). The paired fibronectin type 3 repeats are located at about position 428 through position 535 and about position 536 through about position 642. One pair of cysteines is located at position 436 and position 447, and the second pair is located at position 473 and 488. The "WSXWS box" is located at position 622-626.

Between the first and the second hematopoietin domain (amino acids 339-428, approximately) is a region of unknown functional significance.

The random coil domain ("RC" between the H2 and the transmembrane domain, "TM") is likely to begin at the amino acid following the end of the second hematopoietin domain, and is likely to end at the beginning of the transmembrane domain. This is likely to be from about amino acid 642 through amino acid 839 or 841 (with the transmembrane domain beginning at position 840 (A) or 842 (L)). The intracellular domain ("IC") is likely to begin at position 861 (L), 862 (I), 863 (S) or 864 (H).

The intracellular domain ("IC") contains three regions, or "boxes," thought to participate in signal transduction (two "JAK" boxes and a single "STAT" box, "Box 1", "Box 2", and "Box 3"). With respect to the numbering of the amino acid positions of the "D" form of the OB receptor (Seq. ID No.7, below), box 1 is located at amino acid 871 (F) through 878 (P). Box 2 is located at approximately amino acid number 921 (I) through 931 (K). Box 3 on the "D" form is located at approximately position 1141 through 1144 (amino acids YMPQ, as the "STAT" box is typically a conserved region of "YXXQ" wherein "X" designates any amino acid). The intracellular domain is thought to be responsible for signal transduction. One possible mode of action is via phosphorylation of various residues. See Ihle et al., Cell 84: 331-334 (1996)(Review article, herein incorporated by reference.)

One possible mode of action is that upon ligand binding (here, OB protein binding), the OB receptor dimerizes with another receptor. A kinase ("JAK") binds to box 1, and becomes phosphorylated. (The JAK may already be bound prior to dimerization.) Also, "STATS" bind to box 3 and become phosphorylated on a specific tyrosine. It is thought that this phosphorylation results, probably indirectly, in DNA binding protein production, which results in altered DNA transcription, and therefore altered expression. As seen below in Example 6, one measurement of the capability of an OB receptor to transduce signal is the degree of phosphorylation of JAK/STAT molecules.

The C-terminus region is intracellular (of cell-bound OB receptor). The differences in the C-terminus among members of the present OB receptor family may result in differences in signal transduction among the species. Thus, the present OB receptors include at least the extracellular domain which is important for OB protein ligand binding. Nucleic acids encoding the present OB receptors, vectors, and host cells are also provided for herein.

The extracellular domain may be modified and still retain the function of ligand binding, particularly by one or more of the following modifications: (a) the random coil domain (as indicated above, occuring downstream of the second hematopoietic domain through the beginning of the transmembrane domain) may be deleted (this may be approximately positions 642 through 839 or 841); (b) the "WSXWS" box may be modified by (i) substitution of the first serine with another amino acid, particularly conserved in terms of hydrophobicity and/or charge, such as a glycine; (ii) the last serine may be substituted with another amino acid, such as a threonine; (iii) the first tryptophan may be substituted with another amino acid, for example, a tyrosine.

Human genomic DNA encoding OB receptor protein is also provided herein. The genomic DNA has been localized to human chromosome 1P31, which is believed to correspond to mouse chromosome 4, the location of the mouse db locus.

Tissue distribution analysis demonstrates the presence of OB receptor nucleic acids is fairly ubiquitous, and particularly noted in the liver. It is also observed in the ovary, and heart; and, to a lesser extent, in small intestine, lung, skeletal muscle, kidney, and, to an even lesser extent, spleen, thymus, prostate, testes, placenta and pancreas (Example 2, below). There may also be one or more forms of the OB receptor present in serum, such as soluble OB receptor, which may be complexed to one or more forms of the OB protein.

### Amino Acid Sequences and Compositions

According to the present invention, novel OB protein receptors and DNA sequences coding for all or part of such OB receptors are provided. The present invention provides purified and isolated polypeptide products having part or all of the primary structural conformation (i.e., continuous sequence of amino acid residues) and one or more of the biological properties (e.g., immunological properties and in vitro biological activity) and physical properties (e.g., molecular weight) of naturally-occurring mammalian OB receptor including allelic variants thereof. The term "purified and isolated" herein means substantially free of unwanted substances so that the present polypeptides are useful for an intended purpose. For example, one may have a recombinant human OB receptor substantially free of human proteins or pathological agents. These polypeptides are also characterized by being a product of mammalian cells, or the product of chemical synthetic procedures or of procaryotic or eucaryotic host expression (e.g., by bacterial, yeast, higher plant, insect and mammalian cells in culture) of exogenous DNA sequences obtained by genomic or cDNA cloning or by gene synthesis. The products of expression in typical yeast (e.g., Saccharomyces cerevisiae), insect, or procaryote (e.g., E. coli) host cells are free of association with any mammalian proteins. The products of expression in vertebrate (e.g., non-human mammalian (e.g. COS or CHO) and avian) cells are free of association with any human proteins. Depending upon the host employed, and other factors, polypeptides of the invention may be glycosylated with mammalian or other eucaryotic carbohydrates or may be non-glycosylated. One may modify the nucleic acid so that glycosylation sites are included in the resultant polypeptide. One may choose to partially or fully deglycosylate a glycosylated polypeptide. Polypeptides of the invention may also include an initial methionine amino acid residue (at position -1 with respect to the first amino acid residue of the mature polypeptide).

In addition to naturally-occurring allelic forms of OB receptor, the present invention also describes other OB receptor products such as polypeptide analogs of OB receptor and fragments of OB receptor. Following the procedures of the above noted published application by Alton et al. (WO 83/04053), one can readily design and manufacture genes coding for microbial expression of polypeptides having primary conformations which differ from that herein specified for in terms of the identity or location of one or more residues (e.g., substitutions, terminal and intermediate additions and deletions). Alternately, modifications of cDNA and genomic genes may be readily accomplished by well-known site-directed mutagenesis techniques and employed to generate analogs and derivatives of OB receptor. Such products would share at least one of the biological properties of mammalian OB receptor but may differ in others. As examples, projected products include those which are foreshortened by e.g., deletions; or those which are more stable to hydrolysis (and, therefore, may have more pronounced or longer lasting effects than naturally-occurring); or which have been altered to delete one or more potential sites for glycosylation (which may result in higher activities for yeast-produced products); or which have one or more cysteine residues deleted or replaced by, e.g., alanine or serine residues and are potentially more easily isolated in active form from microbial systems; or which have one or more tyrosine residues replaced by phenylalanine; or have an altered lysine composition (such as those prepared for purposes of derivatization). Described are those polypeptides with amino acid substitutions which are "conservative" according to acidity, charge, hydrophobicity, polarity, size or any other characteristic known to those skilled in the art. See generally, Creighton, Proteins, W.H. Freeman and Company, N.Y., (1984) 498 pp. plus index, passim. One may make changes in selected amino acids so long as such changes preserve the overall folding or activity of the protein, (see Table 1, below). Small amino terminal extensions, such as an amino-terminal methionine residue, a small linker peptide of up to about 20-25 residues, or a small extension that facilitates purification, such as a poly-histidine tract, an antigenic epitope or a binding domain, may also be present. See, in general Ford et al., Protein Expression and Purification 2: 95-107, 1991.

**Table 1**

| Conservative Amino Acid Substitutions | |
|---|---|
| Basic: | arginine |
| | lysine |
| | histidine |
| Acidic: | glutamic acid |
| | aspartic acid |
| Polar: | glutamine |
| | asparaqine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

Also described are polypeptide fragments duplicating only a part of the continuous amino acid sequence or secondary conformations within OB receptor, which fragments may possess one activity of mammalian (particularly human) OB receptor (e.g., immunological activity) and not others (e.g., OB protein binding activity).

Of applicability to OB receptor fragments and polypeptide analogs are reports of the immunological activity of synthetic peptides which substantially duplicate the amino acid sequence extant in naturally-occurring proteins, glycoproteins and nucleoproteins. More specifically, relatively low molecular weight polypeptides have been shown to participate in immune reactions which are similar in duration and extent to the immune reactions of physiologically significant proteins such as viral antigens, polypeptide hormones, and the like. Included among the immune reactions of such polypeptides is the provocation of the formation of specific antibodies in immunologically active animals. See, e.g., Lerner et al., Cell 23: 309-310 (1891); Ross et al., Nature 294: 654-656 (1891); Walter et al., PNAS-USA 77: 5197-5200 (1980); Lerner et al., PNAS-USA, 78: 3403-3407 (1891); Walter et al., PNAS-USA 78: 4882-4886 (1891); Wong et al., PNAS-USA 79: 5322-5326 (1982); Baron et al., Cell 28: 395-404 (1982); Dressman et al., Nature 295: 185-160 (1982); and Lerner, Scientific American 248: 66-74 (1983). See, also, Kaiser et al. Science 223: 249-255 (1984) relating to biological and immunological activities of synthetic peptides which approximately share secondary structures of peptide hormones but may not share their primary structural conformation. The present invention also describes that class of polypeptides coded for by portions of the DNA complementary to the protein-coding strand of the human cDNA or genomic DNA sequences of OB receptor i.e., "complementary inverted proteins" as described by Tramontano et al. Nucleic Acid Res. 12: 5049-5059 (1984). Polypeptides or analogs thereof may also contain one or more amino acid analogs, such as peptidomimetics.

Thus, the present class of OB receptor proteins is those having amino acids (according to Seq. ID No. 1):
(a) 1-896;
(b) 22-896;
(c) 23-896;
(d) 29-896
(e) 22-891;
(f) 23-891;
(g) 29-891;
(h) sequences (e) through (g) having the C-terminal amino acid sequence beginning at position 892 of OB receptor B (Seq. ID No. 3) or C (Seq. ID. No. 5);
(i) amino acids of subparts b, c, d, e, f, g, and (h) lacking a leader sequence, which have an N-terminal methionyl residue, and
(j) a chemically modified derivative of any of subparts (a) through (h)

As set forth above, one may prepare soluble receptor by elimination of the transmembrane and intracellular regions. Examples of soluble receptors include those set forth in Seq. ID Nos. 10 and 13. What is thought to be a native, secreted form of a soluble human OB receptor is also described herein. This form of OB receptor protein has an amino acid sequence selected from among (according to Seq. ID No. 13):
(a) amino acids 1-804;
(b) amino acids 22-804;
(c) amino acids 23-804;
(d) amino acids 29-804; and,
(e) amino acids of subparts (b), (c) or (d) having an N-terminal methionyl residue.

In addition, since the C-terminus region of the above polyeptides diverges at position 892 (with respect to Seq. ID Nos. 1, 3, 5, 7 and 13) one may desire to prepare only the polypeptides which are divergent:
(a) those having only amino acids 892-896 of Seq. ID No. 1;
(b) those having only amino acids 892-904 of Seq. ID No. 3;
(c) those having only amino acids 892-958 of Seq. ID No. 5;
(d) those having only amino acids 892-1165 of Seq. ID No. 7; and,
(e) those having only amino acids 799-804 of Seq. ID No. 13.

The above polypeptides which have an extracellular domain may be modified, as indicated above, and still retain the function of ligand binding. Such modification may include one or more of the following:
(a) the random coil domain (as indicated above, occuring downstream of the second hematopoietic domain through the beginning of the transmembrane domain) may be deleted (this may be approximately positions 642 through 839 or 841);
(b) the "WSXWS" box may be modified by (i) substitution of the first serine with another amino acid, particularly conserved in terms of hydrophobicity and/or charge, such as a glycine; (ii) the last serine may be substituted with another amino acid, such as a threonine; (iii) the first tryptophan may be substituted with another amino acid, for example, a tyrosine.

Thus, the present polypeptides include (according to the numbering of Seq. ID No. 7):
(a) 1-896;
(b) 22-896;
(c) 23-896;
(d) 29-896
(e) 22-891;
(f) 23-891;
(g) 29-891;
(h) sequences (e) through (g) having the C-terminal amino acids selected from the C-terminal amino acids 892-904 of OB receptor B (Seq. ID No. 3), or amino acids 892-958 of OB receptor) C (Seq. ID. No. 5) ;
(i) amino acids of subparts b, c, d, e, f, g, and (h) lacking a leader sequence, and which have an N-terminal methionyl residue; and
(j) amino acids of subparts (a) through (i) above having at least one of the following modifications:
   (i) deletion of all or part of the random coil domain sequence selected from
      (a) 640 through 839 (using the numbering according to Seq. ID No. 1);
      (b) 641 through 839;
      (c) 642 through 839;
      (d) 640 through 841;
      (e) 641 through 841; and
      (f) 642 through 841;
      and,
   (ii) modification of a "WSXWS" sequence which
      (a) substitution of the first serine with another amino acid, particularly conserved in terms of hydrophobicity and/or charge, such as a glycine;
      (b) substition of the last serine with another amino acid, such as a threonine; and
      (c) substitution of the first tryptophan with another amino acid, for example, a tyrosine.

One may modify the OB receptor to create a fusion molecule with other peptide sequence. For example, if one desired to "tag" the OB receptor with an immunogenic peptide, one could construct a DNA which would result in such fusion protein. The tag may be at the N-terminus. Also, since it is apparent that the C-terminus is not necessary for ligand binding activity, one may chemically modify the C-terminus of, for example, a soluble OB receptor. One may desire, for example, a preparation whereby one or more polymer molecules such as polyethylene glycol molecules are attached. Thus, another aspect of the present invention is chemically modified OB receptor protein (also further described infra).

An example of such "tag" is provided herein using the C-terminus of a recombinant soluble OB receptor. Seq. ID No. 12 provides a "FLAG-tag" version of such soluble OB receptor (the nucleic acid sequence is provided, which may be transcribed to prepare the polypeptide). Such "FLAG-tag" may also be attached to the N-terminus or other region of an OB receptor protein. This type of "tagging" is useful to bind the protein using reagents, such as antibodies, which are selective for such tag. Such binding may be for detection of the location or amount of protein, or for protein capturing processes where, for example, an affinity column is used to bind the tag, and thus the desired protein. Other types of detectable labels, such as radioisotopes, light-emitting (e.g., fluorescent or phosporescent compounds), enzymatically cleavable, detectable antibody (or modification thereof), or other substances may be used for such labelling of the present proteins. Detecting protein via use of the labels may be useful for identifying the presence or amount of OB receptor protein or a compound containing such protein (e.g., OB protein complexed to OB receptor). Moreover, such labelled protein may be useful for distinguishing exogenous OB receptor protein from the endogenous form.

### Nucleic Acids

Novel nucleic acid sequences of the invention include sequences useful in securing expression in procaryotic or eucaryotic host cells of polypeptide products having at least a part of the primary structural conformation and one or more of the biological properties of recombinant human OB receptor. The nucleic acids may be purified and isolated, so that the desired coding region is useful to produce the present polypeptides, for example, or for diagnostic purposes, as described more fully below. DNA sequences of the invention specifically comprise: (a) any of the DNA sequences set forth in Seq. ID No. 2, 4, 6, (and complementary strands); (b) a DNA sequence which hybridizes (under hybridization conditions disclosed in the cDNA library screening section below, using the 300 bp PCR fragment as described to selectively hybridize to a cDNA encoding an OB receptor protein in a human liver cDNA library, or equivalent conditions or more stringent conditions) to the DNA sequence in subpart (a) or to fragments thereof; and (c) a DNA sequence which, but for the degeneracy of the genetic code, would hybridize to the DNA sequence in subpart (a). Specifically comprehended in parts (b) and (c) are genomic DNA sequences encoding allelic variant forms of human OB receptor and/or encoding OB receptor from other mammalian species, and manufactured DNA sequences encoding OB receptor, fragments of OB receptor, and analogs of OB receptor which DNA sequences may incorporate codons facilitating transcription and translation of messenger RNA in microbial hosts. Such manufactured sequences may readily be constructed according to the methods of Alton et al., PCT published application WO 83/04053.

Genomic DNA, such as that of Seq. ID No. 9, encoding the present OB receptors may contain additional non-coding bases, or introns, and such genomic DNAs are obtainable by hybridizing all or part of the cDNA, illustrated in Seq. ID Nos. 2, 4, 6, 8, 11, and 14 to a genomic DNA source, such as a human genomic DNA library. Such genomic DNA will encode functional OB receptor polypeptide; however, use of the cDNAs may be more practicable in that, since only the coding region is involved, recombinant manipulation is facilitated. The intron/exon location of genomic DNA is set forth in Seq. ID No. 9, infra.

Nucleic acid sequences include the incorporation of codons which enhance expression by selected nonmammalian hosts; the provision of sites for cleavage by restriction endonuclease enzymes; and the provision of additional initial, terminal or intermediate DNA sequences which facilitate construction of cloning and/or expression vectors.

The present invention also describes DNA sequences coding for polypeptide analogs or derivatives of OB receptor which differ from naturally-occurring forms in terms as described above. The leader sequence DNA may be substituted with another leader sequence for ease in expression or for other purposes.

Also, one may prepare antisense nucleic acids against the present DNAs. Such antisense nucleic acids may be useful in modulating the effects of OB receptor protein in vivo. For example, one may prepare an antisense nucleic acid which effectively disables the ability of a cell to produce OB receptor by binding to the nucleic acid which encodes such OB receptor.

DNA sequences of the invention are also suitable materials for use as labeled probes in isolating human genomic DNA encoding OB receptor, as mentioned above, and related proteins as well as cDNA and genomic DNA sequences of other mammalian species. DNA sequences may also be useful in various alternative methods of protein synthesis (e.g., in insect cells) or, as described infra, in genetic therapy in humans and other mammals. DNA sequences of the invention are expected to be useful in developing transgenic mammalian species which may serve as eucaryotic "hosts" for production of OB receptor and OB receptor products in quantity. See, generally, Palmiter et al., Science 222: 809-814 (1983).

### Vectors and Host Cells

According to another aspect of the present invention, the DNA sequences described herein which encode OB receptor polypeptides are valuable for the information which they provide concerning the amino acid sequence of the mammalian protein which have heretofore been unavailable. Put another way, DNA sequences provided by the invention are useful in generating new and useful viral and circular plasmid DNA vectors, new and useful transformed and transfected procaryotic and eucaryotic host cells (including bacterial cells, yeast cells, insect cells, and mammalian cells grown in culture), and new and useful methods for cultured growth of such host cells capable of expression of OB receptor and its related products.

The DNA provided herein (or corresponding RNAs) may also be used for gene therapy for, example, treatment of conditions characterized by the overexpression of OB protein, such as anorexia or cachexia. Alternatively, gene therapy may be used in cases where increased sensitivity to OB protein is desired, such as in cases where an individual has a condition characterized by OB protein receptors defective in ability to bind or retain the binding of OB protein. Currently, vectors suitable for gene therapy (such as retroviral or adenoviral vectors modified for gene therapy purposes and of purity and pharmaceutical acceptability) may be administered for delivery into the lung, for example. Such vectors may incorporate nucleic acid encoding the present polypeptides for expression in a desired location. Gene therapy may involve more than one gene for a desired protein or different desired proteins.

Alternatively, one may use no vector so as to facilitate relatively stable presence in the host. For example, homologous recombination of a DNA as provided herein or of a suitable transcription or translation control region may facilitate integration into or expression from a host genome. (This may be performed for production purposes as well, e.g., U.S. Patent No. 5,272,071 and WO 91/09955.) The nucleic acid may be placed within a pharmaceutically acceptable carrier to facilitate cellular uptake, such as a lipid solution carrier (e.g., a charged lipid), a liposome, or polypeptide carrier (e.g., polylysine). A review article on gene therapy is Verma, Scientific American, November 1990, pages 68-84 which is herein incorporated by reference.

Thus, the present invention provides for a population of cells expressing an OB receptor of the present OB receptor family. Such cells are suitable for transplantation or implantation into an individual for therapeutic purposes. For example, one may prepare a population of cells to overexpress OB receptor (such as one identified in the Sequence ID's or otherwise denoted herein), or to express a desired form of OB receptor, such as one which is particularly sensitive to OB protein (i.e., a form which has a desired capacity for signal transduction). One may then implant such cells into an individual to increase that individual's sensitivity to OB protein. Such cells may, for example, be liver cells, bone marrow cells, or cells derived from umbillical cord. Alternatively, one may wish to use overexpressing circulating cells such as blood progenitor cells, T cells or other blood cells. For humans, human cells may be used. Cells may be in the form of tissue. Such cells may be cultured prior to transplantation or implantation. Such OB receptor overexpression, or expression of particularly sensitive forms of OB receptor may be accomplished by, for example, altering the regulatory mechanism for expression of OB receptor, such as using homologous recombination techniques as described supra. Thus, provided is a population of host cells modified so that expression of endogenous OB receptor DNA is enhanced.

The cells to be transferred to the recipient may be cultured using one or more factors affecting the growth or proliferation of such cells if appropriate. Hematopoietic factors may be used in culturing hematopoietic cells. Such factors include G-CSF, EPO, MGDF, SCF, Flt-3 ligand, interleukins (e.g., IL1-IL13), GM-CSF, LIF, and analogs and derivatives thereof as available to one skilled in the art.

Nerve cells, such as neurons or glia, may also be used, and these may be cultured with neurotrophic factors such as BDNF, CNTF, GDNF, NT3, or others.

There may be a co-gene therapy involving the transplantation of cells expressing more than one desired protein. For example, cells expressing OB receptor protein may be used in conjunction, simultaneously or in serriatim with cells expressing OB protein.

For gene therapy dosages, one will generally use between one copy and several thousand copies of the present nucleic acid per cell, depending on the vector, the expression system, the age, weight and condition of the recipient and other factors which will be apparent to those skilled in the art. The cellular delivery of such protein may be designed to last for a selected period of time, such as a period of days, weeks, months or years. At the end of the effective time period, the recipient of such transformed cells may receive another "dose" (e.g., transplantation of cells). Cells may be selected for their lifespan, their time period of expression of the desired protein, or their ability to be reisolated from an individual (i.e., for blood cells, leukaphoresis may be used to retrieve transformed cells using markers present on the cell surface). Vectors may be similiarly designed using, for example, viruses which have a known period of expression of DNAs contained therein.

The desired cells or vectors may be stored using techniques, such as freezing, available to those in the art.

Thus, the present invention also describes a method for administering OB receptor protein to an individual, wherein the source of said OB receptor protein is selected from (i) a population of cells expressing OB receptor protein and (ii) a population of vectors expressing OB receptor protein. Said OB receptor protein may be selected from among those described herein. Said vectors may be virus vectors capable of infecting human cells. Said cells may be selected from among tissue or individual cells. Said individual cells may be selected from among adipocytes, fibroblasts, bone marrow cells, peripheral blood progenitor cells, red blood cells, and white blood cells, including T cells and nerve cells. Said population of cells or vectors may be co-administered with a population of cells or vectors which express OB protein or another desired protein. Said cells or vectors may be stored for use in an individual. Storage may be by freezing

### Complexes

In addition to the OB receptor protein as described herein, one may prepare complexes of OB receptor protein and OB protein, analog or derivative.

The OB protein may be selected from those described in PCT publication WO 96/05309, Figure 3 of that publication (Seq. ID No. 4, as cited therein) depicts the full deduced amino acid sequence derived for the human OB gene. The amino acids are numbered from 1 to 167. A signal sequence cleavage site is located after amino acid 21 (Ala) so that the mature protein extends from amino acid 22 (Val) to amino acid 167 (Cys). For the present disclosure, a different numbering is used herein, where the amino acid position 1 is the Valine residue which is at the beginning of the mature protein.

Generally, the OB protein for use will be capable of complexing to the OB protein receptor selected. Thus, one may empirically test the binding capability (to all or part of the extracellular domain of the OB receptor as indicated above) to determine which OB protein forms may be used. Generally, modifications generally applicable as indicated above for OB receptor protein may also be applied here. As set forth in WO 96 05309, OB protein in its native form, or fragments (such as enzyme cleavage products) or other truncated forms, analogs, and derivatives all retain biological activity. Such forms may be used so long as the form binds to at least a portion of the extracellular domain of the present OB receptor proteins.

An effective amount of an OB protein, analog or derivative thereof may be selected from among according to the amino acid sequence as presented in PCT WO 96/05309, Figure 3 numbered so that the first amino acid of the mature protein is number 1:
(a) the amino acid sequence 1-146, optionally lacking a glutaminyl residue at position 28, and further optionally having a methionyl residue at the N-terminus;
(b) an amino acid sequence of subpart

(a) having a different amino acid substituted in one or more of the following positions: 4, 8, 32, 33, 35, 48, 50, 53, 60, 64, 66, 67, 68, 71, 74, 77, 78, 89, 97, 100, 102, 105, 106, 107, 108, 111, 112, 118, 136, 138, 142, and 145;
(c) a truncated OB protein analog selected from among: (using the numbering of subpart (a) above):
   (i) amino acids 98-146
   (ii) amino acids 1-32
   (iii) amino acids 1-35
   (iv) amino acids 40-116
   (v) amino acids 1-99 and 112-146
   (vi) amino acids 1-99 and 112-146 having one or more of amino acids 100-111 sequentially placed between amino acids 99 and 112; and,
   (vii) the truncated OB analog of subpart (i) having one or more of amino acids 100, 102, 105, 106, 107, 108, 111, 112, 118, 136, 138, 142, and 145 substituted with another amino acid;
   (viii) the truncated analog of subpart (ii) having one or more of amino acids 4, 8 and 32 substituted with another amino acid;
   (ix) the truncated analog of subpart (iv) having one or more of amino acids 50, 53, 60, 64, 66, 67, 68, 71, 74, 77, 78, 89, 97, 100, 102, 105, 106, 107, 108, 111 and 112 replaced with another amino acid;
   (x) the truncated analog of subpart (v) having one or more of amino acids 4, 8, 32, 33, 35, 48, 50, 53, 60, 64, 66, 67, 68, 71, 74, 77, 78, 89, 97, 112, 118, 136, 138, 142, and 145 replaced with another amino acid;
   (xi) the truncated analog of subpart (vi) having one or more of amino acids 4, 8,32, 33, 35, 48, 50, 53, 60, 64, 66, 67, 68, 71, 74, 77, 78, 89, 97, 100, 102, 105, 106, 107, 108, 111, 112, 118, 136, 138, 142, and 145 replaced with another amino acid;
   (xii) the truncated analog of any of subparts (i)-(xi) having an N-terminal methionyl residue; and
(d) the OB protein or analog derivative of any of subparts (a) through (c) comprised of a chemical moiety connected to the protein moiety;
(e) a derivative of subpart (d) wherein said chemical moiety is a water soluble polymer moiety;
(f) a derivative of subpart (e) wherein said water soluble polymer moiety is polyethylene glycol;
(g) A derivative of subpart (f) wherein said water soluble polymer moiety is a polyamino acid moiety;
(h) a derivative of subpart (g) wherein said water soluble polymer moiety is attached at solely the N-terminus of said protein moiety;
(i) an OB protein, analog or derivative of any of subparts (a) through (h) in a pharmaceutically acceptable carrier.

OB proteins, analogs and related molecules are also reported in the following publications; however, no representation is made with regard to the activity of any composition reported:
U.S.Patent Nos. 5,521,283; 5,532,336; 5,552,522; 5,552,523; 5,552,524; 5,554,727; 5,559,208; 5,563,243; 5,563,244; 5,563,245; 5,567,678; 5,567,803; 5,569,744; 5,569,743 (all assigned to Eli Lilly and Company);
PCT WO96/23517; WO96/23515; WO96/23514;
WO96/24670; WO96/23513; WO96/23516;
WO96/23518; WO96/23519; WO96/23520;
WO96/23815; WO96/24670; WO96/27385 (all assigned to Eli Lilly and Company);
PCT WO96/22308 (assigned to Zymogenetics);
PCT WO96/29405 (assigned to Ligand Pharmaceuticals, Inc.);
PCT WO96/31526 (assigned to Amyin Pharmaceuticals, Inc.);
PCT WO96/34885 (assigned to Smithkline Beecham PLC);
PCT WO96/35787 (assigned to Chiron);
EP 0 725 079 (assigned to Eli Lilly and Company);
EP 0 725 078 (assigned to Eli Lilly and Company);
EP 0 736 599 (assigned to Takeda);
EP 0 741 187 (assigned to F. Hoffman LaRoche).

To the extent these references provide for useful OB proteins or analogs or derivatives thereof, or associated compositions or methods, such compositions and/or methods may be used in conjunction with the present OB receptor proteins, such as for coadministration (together or separately, in a selected dosage schedule) or by complexing compositions to the present OB protein receptors.

### Derivatives and Formulations

The present OB protein receptor and/or OB protein (herein the term "protein" is used to include "peptide" and OB protein or receptor analogs, such as those recited infra, unless otherwise indicated) may also be derivatized by the attachment of one or more chemical moieties to the protein moiety. If the present pharmaceutical compositions contain as the active ingredient a complex of OB protein receptor and OB protein, one or both of such proteins may be derivatized. The chemically modified derivatives may be further formulated for intraarterial, intraperitoneal, intramuscular, subcutaneous, intravenous, oral, nasal, pulmonary, topical or other routes of administration. Chemical modification of biologically active proteins has been found to provide additional advantages under certain circumstances, such as increasing the stability and circulation time of the therapeutic protein and decreasing immunogenicity. See U.S. Patent No. 4,179,337, Davis et al., issued December 18, 1979. For a review, see Abuchowski et al., in Enzymes as Drugs. (J.S. Holcerberg and J. Roberts, eds. pp. 367-383 (1891)). A review article describing protein modification and fusion proteins is Francis, Focus on Growth Factors 3: 4-10 (May 1992) (published by Mediscript, Mountview Court, Friern Barnet Lane, London N20, OLD, UK).

Preferably, for therapeutic use of the end-product preparation, the chemical moiety for derivatization will be pharmaceutically acceptable. A polymer may be used. One skilled in the art will be able to select the desired polymer based on such considerations as whether the polymer/protein conjugate will be used therapeutically, and if so, the desired dosage, circulation time, resistance to proteolysis, and other considerations. For the present proteins and peptides, the effectiveness of the derivatization may be ascertained by administering the derivative, in the desired form (i.e., by osmotic pump, or by injection or infusion, or, further formulated for oral, pulmonary or nasal delivery, for example), and observing biological effects as described herein.

The chemical moieties suitable for derivatization may be selected from among various water soluble polymers. The polymer selected should be water soluble so that the protein to which it is attached so that it is miscible in an aqueous environment, such as a physiological environment. The water soluble polymer may be selected from the group consisting of, for example, polyethylene glycol, copolymers of ethylene glycol/propylene glycol, carboxymethylcellulose, dextran, polyvinyl alcohol, polyvinyl pyrolidone, poly-1, 3-dioxolane, poly-1,3,6-trioxane, ethylene/maleic anhydride copolymer, polyaminoacids (either homopolymers or random or non-random copolymers (see supra regarding fusion molecules), and dextran or poly(n-vinyl pyrolidone)polyethylene glycol, propylene glycol homopolymers, polypropylene oxide/ethylene oxide co-polymers, polyoxyethylated polyols, polystyrenemaleate and polyvinyl alcohol. Polyethylene glycol propionaldenhyde may have advantages in manufacturing due to its stability in water.

Fusion proteins may be prepared by attaching polyaminoacids to the OB protein receptor or OB protein (or analog or complex) moiety. For example, the polyamino acid may be a carrier protein which serves to increase the circulation half life of the protein. For the present therapeutic or cosmetic purposes, such polyamino acid should be those which do not create neutralizing antigenic response, or other adverse response. Such polyamino acid may be selected from the group consisting of serum album (such as human serum albumin), an antibody or portion thereof (such as an antibody constant region, sometimes called "F_{c}") or other polyamino acids. As indicated below, the location of attachment of the polyamino acid may be at the N-terminus of the OB protein moiety, or other place, and also may be connected by a chemical "linker" moiety to the OB protein.

The polymer may be of any molecular weight, and may be branched or unbranched. For polyethylene glycol, the preferred molecular weight is between about 2 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog).

The number of polymer molecules so attached may vary, and one skilled in the art will be able to ascertain the effect on function. One may mono-derivatize, or may provide for a di-, tri-, tetra- or some combination of derivatization, with the same or different chemical moieties (e.g., polymers, such as different weights of polyethylene glycols). The proportion of polymer molecules to protein (or peptide) molecules will vary, as will their concentrations in the reaction mixture. In general, the optimum ratio (in terms of efficiency of reaction in that there is no excess unreacted protein or polymer) will be determined by factors such as the desired degree of derivatization (e.g., mono, di-, tri-, etc.), the molecular weight of the polymer selected, whether the polymer is branched or unbranched, and the reaction conditions.

The chemical moieties should be attached to the protein with consideration of effects on functional or antigenic domains of the protein. There are a number of attachment methods available to those skilled in the art. E.g., EP 0 401 384 (coupling PEG to G-CSF), see also Malik et al., Exp. Hematol. 20: 1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule (or other chemical moiety) may be bound. The amino acid residues having a free amino group may include lysine residues and the N-terminal amino acid residue. Those having a free carboxyl group may include aspartic acid residues, glutamic acid residues, and the C-terminal amino acid residue. Sulfhydrl groups may also be used as a reactive group for attaching the polyethylene glycol molecule(s) (or other chemical moiety). Preferred for therapeutic manufacturing purposes is attachment at an amino group, such as attachment at the N-terminus or lysine group. Attachment at residues important for receptor binding should be avoided if receptor binding is desired.

One may specifically desire N-terminally chemically modified protein. Using polyethylene glycol as an illustration of the present compositions, one may select from a variety of polyethylene glycol molecules (by molecular weight, branching, etc.), the proportion of polyethylene glycol molecules to protein molecules in the reaction mix, the type of pegylation reaction to be performed, and the method of obtaining the selected N-terminally pegylated protein. The method of obtaining the N-terminally pegylated preparation (i.e., separating this moiety from other monopegylated moieties if necessary) may be by purification of the N-terminally pegylated material from a population of pegylated protein molecules. Selective N-terminal chemical modification may be accomplished by reductive alkylation which exploits differential reactivity of different types of primary amino groups (lysine versus the N-terminal) available for derivatization in a particular protein. See PCT WO 96/11953. Under the appropriate reaction conditions, substantially selective derivatization of the protein at the N-terminus with a carbonyl group containing polymer is achieved. For example, one may selectively N-terminally pegylate the protein by performing the reaction at a pH which allows one to take advantage of the pKₐ differences between the e-amino group of the lysine residues and that of the a-amino group of the N-terminal residue of the protein. By such selective derivatization, attachment of a polymer to a protein is controlled: the conjugation with the polymer takes place predominantly at the N-terminus of the protein and no significant modification of other reactive groups, such as the lysine side chain amino groups, occurs. Using reductive alkylation, the polymer may be of the type described above, and should have a single reactive aldehyde for coupling to the protein. Polyethylene glycol propionaldehyde, containing a single reactive aldehyde, may be used.

An N-terminally chemically modified derivative is preferred (over other forms of chemical modification) for ease in production of a therapeutic. N-terminal chemical modification ensures a homogenous product as characterization of the product is simplified relative to di-, tri- or other multi-derivatized products. The use of the above reductive alkylation process for preparation of an N-terminally chemically modified product is preferred for ease in commercial manufacturing.

In yet another aspect of the present invention, provided are methods of using pharmaceutical compositions of the proteins, and derivatives. Such pharmaceutical compositions may be for administration by injection, or for oral, pulmonary, nasal, transdermal or other forms of administration. In general, comprehended by the invention are pharmaceutical compositions comprising effective amounts of protein or derivative products of the invention together with pharmaceutically acceptable diluents, preservatives, solubilizers, emulsifiers, adjuvants and/or carriers. Such compositions include diluents of various buffer content (e.g., Tris-HCl, acetate, phosphate), pH and ionic strength; additives such as detergents and solubilizing agents (e.g., Tween 80, Polysorbate 80), anti-oxidants (e.g., ascorbic acid, sodium metabisulfite), preservatives (e.g., Thimersol, benzyl alcohol) and bulking substances (e.g., lactose, mannitol); incorporation of the material into particulate preparations of polymeric compounds such as polylactic acid, polyglycolic acid, etc. or into liposomes. See, e.g., PCT WO96/29989, Collins et al., "Stable protein: phospholipid compositions and methods," published October 3, 1996 . Hylauronic acid may also be used, and this may have the effect of promoting sustained duration in the circulation. Such compositions may influence the physical state, stability, rate of in vivo release, and rate of in vivo clearance of the present proteins and derivatives. See, e.g., Remington's Pharmaceutical Sciences, 18th Ed. (1990, Mack Publishing Co., Easton, PA 18042) pages 1435-1712. The compositions may be prepared in liquid form, or may be in dried powder, such as lyophilized form. Implantable sustained release formulations are also contemplated, as are transdermal formulations.

Specifically contemplated are oral dosage forms of the above derivatized proteins. Protein may be chemically modified so that oral delivery of the derivative is efficacious. Generally, the chemical modification contemplated is the attachment of at least one moiety to the protein (or peptide) molecule itself, where said moiety permits (a) inhibition of proteolysis; and (b) uptake into the blood stream from the stomach or intestine. Also desired is the increase in overall stability of the protein and increase in circulation time in the body. See PCT W095/21629, Habberfield, "Oral Delivery of Chemically Modified Proteins" (published August 17, 1995), and U.S. Patent No. 5,574,018, Habberfield et al., "Conjugates of Vitamin B12 and Proteins," issued November 12, 1996 .

Also contemplated herein is pulmonary delivery of the present protein, or derivative thereof. The protein (derivative) is delivered to the lungs of a mammal while inhaling and traverses across the lung epithelial lining to the blood stream. See, PCT WO94/20069, Niven et al., "Pulmonary administration of granulocyte colony stimulating factor," published September 15, 1994 .

Nasal delivery of the protein (or analog or derivative) is also contemplated. Nasal delivery allows the passage of the protein to the blood stream directly after administering the therapeutic product to the nose, without the necessity for deposition of the product in the lung. Formulations for nasal delivery include those with absorption enhancing agents, such as dextran or cyclodextran. Delivery via transport across other mucous membranes is also contemplated.

### Dosages

One skilled in the art will be able to ascertain effective dosages by administration and observing the desired therapeutic effect. Preferably, the formulation of the molecule or complex in a pharmaceutical composition will be such that between about .10 µg/kg/day and 10 mg/kg/day will yield the desired therapeutic effect. The effective dosages may be determined using diagnostic tools over time. For example, a diagnostic for measuring the amount of OB protein or OB receptor protein in the blood (or plasma or serum) may first be used to determine endogenous levels of OB protein (or receptor). Such diagnostic tool may be in the form of an antibody assay, such as an antibody sandwich assay. The amount of endogenous OB receptor protein (such as soluble receptor) is quantified initially, and a baseline is determined. The therapeutic dosages are determined as the quantification of endogenous and exogenous OB receptor protein (that is, protein, analog or derivative found within the body, either self-produced or administered) is continued over the course of therapy. The dosages may therefore vary over the course of therapy, with a relatively high dosage being used initially, until therapeutic benefit is seen, and lower dosages used to maintain the therapeutic benefits.

During an initial course of therapy of an obese person, dosages may be administered whereby weight loss and concomitant fat tissue decrease increase is achieved. Once sufficient weight loss is achieved, a dosage sufficient to prevent re-gaining weight, yet sufficient to maintain desired weight or fat mass may be administered. These dosages can be determined empirically, as the effects of OB protein are reversible. E.g., Campfield et al., Science 269: 546-549 (1995) at 547. Thus, if a dosage resulting in weight loss is observed when weight loss is not desired, one would administer a lower dose, yet maintain the desired weight.

### Therapeutic Compositions and Methods

The present OB receptor proteins, alone, or in combination with an OB protein, and nucleic acids may be used for methods of treatment, or for methods of manufacturing medicaments for treatment. Such treatment includes conditions characterized by excessive production of OB protein, wherein the present OB receptors, particularly in soluble form, may be used to complex to and therefore inactivate such excessive OB protein. Or, such OB receptor protein, particularly in soluble form, may act to protect the activity of OB protein. While not wishing to be bound by theory, one may postulate that OB protein receptor agonist activity may be accomplished by a protective effect achieved when OB protein receptor (particularly soluble receptor) is complexed to OB protein. Such effect may prolong the serum half life of OB protein in vivo. Such treatments may be accomplished by preparing soluble receptor (e.g., use of an extracellular domain as described supra) and administering such composition to an individual in need thereof or by preparation of a population of cells containing or expressing such OB receptor, and transplanting such cells into the individual in need thereof.

The present OB receptors may also be used for treatment of those having defective OB receptors. For example, one may treat an individual having defective OB receptors by preparation of a population of cells containing such non-defective OB receptor, and transplanting such cells into an individual. Or, an individual may have an inadequate number of OB receptors, and cells containing such receptors may be transplanted in order to increase the number of OB receptors available to an individual.

The present OB receptor proteins and related compositions such as OB receptor protein/OB protein complex, provide for weight loss, fat loss, increase in lean mass, increase in insulin sensitivity, increase in overall strength, increase in red blood cells (and oxygenation in the blood), decrease in bone resportion or osteoporosis, decreased or maintained serum cholesterol level, decreased or maintained triglyceride (LDL or VLDL) levels, prevention or reduction in arterial plaque formation, treatment of hypertension, and prevention or reduction of gall stone formation. As body fat composition may be correlated with certain types of cancers, the present compositions may be useful for the prevention or amelioration of certain types of cancers. The present invention also includes methods for manufacture of a medicament for use in conjunction with the cosmetic/therapeutic conditions described herein, containing at least one of the present compositions.

The present compositions and methods may be used in conjunction with other medicaments, such as those useful for the treatment of diabetes (e.g., insulin or analogs thereof, thiazolidinediones or other antihyperglycemic agents, and possibly amylin or antagonists there of), cholesterol and blood pressure lowering medicaments (such as those which reduce blood lipid levels or other cardiovascular medicaments), and activity increasing medicaments (e.g., amphetamines). Appetite suppressants may also be used (such as serotonin modulators and neuropeptide Y antagonists). Such administration may be simultaneous or may be in seriatim.

In addition, the present methods may be used in conjunction with surgical procedures, such as cosmetic surgeries designed to alter the overall appearance of a body (e.g., liposuction or laser surgeries designed to reduce body mass, or implant surgeries designed to increase the appearance of body mass). The health benefits of cardiac surgeries, such as bypass surgeries or other surgeries designed to relieve a deleterious condition caused by blockage of blood vessels by fatty deposits, such as arterial plaque, may be increased with concomitant use of the present compositions and methods. Methods to eliminate gall stones, such as ultrasonic or laser methods, may also be used either prior to, during or after a course of the present therapeutic methods. Furthermore, the present methods may be used as an adjunct to surgeries or therapies for broken bones, damaged muscle, or other therapies which would be improved by an increase in lean tissue mass.

In yet another aspect, the present invention provides for methods of manufacture of a medicament for the treatment of obesity, type II diabetes, excess blood lipid, or cholesterol levels, increasing sensitivity to insulin, increasing lean mass, and other conditions as set forth above. Also provided are solely cosmetic treatments for individuals wishing to improve appearance by weight loss, and more specifically, loss of fat deposits, even in the absence of any therapeutic benefit.

### Diagnostic Compositions and Methods

As indicated supra, polypeptide products of the invention may be "labeled" by association with a detectable marker substance (e.g., radiolabeled with ¹²⁵I, fluorescent, chemiluminescent, enzyme) to provide reagents useful in detection and quantification of OB receptor (or complexes) in solid tissue and fluid samples such as blood or urine. Nucleic acid products of the invention may also be labeled with detectable markers (such as radiolabels and non-isotopic labels such as biotin) and employed in hybridization processes to locate the human OB receptor gene position and/or the position of any related gene family in a chromosomal map. Nucleic acid sequences which selectively bind the human OB receptor gene are useful for this purpose. They may also be used for identifying human OB receptor gene disorders at the DNA level and used as gene markers for identifying neighboring genes and their disorders. Such nucleic acid sequences may be sued for detection or measurement of OB receptor mRNA level from a biological sample. Contemplated herein are kits containing such labelled materials.

The protein and/or nucleic acids provided herein may also be embodied as part of a kit or article of manufacture. Contemplated is an article of manufacture comprising a packaging material and one or more preparations of the presently provided compositions. Such packaging material will comprise a label indicating that the protein or nucleic acid preparation is useful for detecting and/or quantifying the amount of OB receptor in a biological sample, or OB receptor defects in a biological sample. As such, the kit may optionally include materials to carry out such testing, such as reagents useful for performing DNA or RNA hybridization analysis, or PCR analysis on blood, urine, or tissue samples.

Further described are selective binding molecules, such as monoclonal antibodies selectively binding OB receptor. The hybridoma technique described originally by Kohler and Milstein Eur. J. Immunol. 6, 511-519 (1976) has been widely applied to produce hybrid cell lines that secrete high levels of monoclonal antibodies against many specific antigens. Recombinant antibodies, (see Huse et al., Science 246: 1275 (1989)) may also be prepared. Such recombinant antibodies may be further modified, such as by modification of complementarity determining regions to increase or alter affinity, or "humanizing" such antibodies. Such antibodies may be incorporated into a kit for diagnostic purposes, for example. A diagnostic kit may be employed to determine the location and/or amount or OB receptor of an individual. Diagnostic kits may also be used to determine if an individual has receptors which bind OB protein, or those which, to varying degrees, have reduced binding capacity or ability. As stated infra, such antibodies may be prepared using immunogenic portions of an OB receptor protein. Such selective binding molecules may themselves be alternatives to OB protein, and may be formulated for pharmaceutical composition.

Such proteins and/or nucleic acids may be used for tissue distribution assays (for example, as provided in the working example below) or for other assays to determine the location of OB receptor.

The present OB receptor protein family may be used in methods to obtain OB protein analogs, mimetics or small molecules. One would simply prepare a desired OB receptor protein, particularly one with capability of binding to native OB protein, and assay the test molecule, which may be labelled with a detectable label substance, for ability to bind to such receptor. Other parameters, such as affinity, and location of binding, may also be ascertained by methods available to those skilled in the art. For example, one could use portions of the present OB receptors, particularly portions in the extracellular domain which are necessary for ligand binding, to determine the location of such binding. One could prepare OB receptors which have various truncations or deletions of regions of the extracellular domain which could be used to determine the location of test molecule binding. One could use an OB receptor known to be defective in native OB binding, such as potentially one from an individual having such defective receptors, and use this as the basis for ascertaining OB protein which would be effective to result in desired biological activity (i.e., weight loss, reduction in blood dyslipidemias or lowering of cholesterol levels, reduction in incidence or severity of diabetes). Other uses include solely cosmetic uses for alteration of body appearance, particularly the removal of fat.

The present OB receptor protein or nucleic acids may also be useful to identify substances which "up-regulate" OB protein or receptor. For instance, the temporal expression of OB receptor in vivo may be useful to determine if an administered substance causes an increase or decrease in OB receptor. One may conclude that an increase in OB receptor expression results in modultion of weight or lipid metabolism.

The divergence in the C-terminus may represent OB receptors with different signal transduction abilities. Therefore the different receptor family members may be used for different assays, depending on the type of signal transduction observed. It is thought that at least a portion of the intracellular domain is necessary for signal transduction (see supra).

The following examples are offered to more fully illustrate the invention, but are not to be construed as limiting the scope thereof.

### EXAMPLE 1: IDENTIFICATION OF HUMAN OB RECEPTOR PROTEIN

Human OB receptor protein DNA was identified in a human liver cDNA library in two steps. The first step used two primers in polymerase chain reaction (PCR) to amplify a selected 300 base pair region from the human liver cDNA library. The second step used the PCR fragment as a probe to screen the human liver cDNA library. Thirteen clones were obtained, but these were incomplete at the 5' end. A procedure was performed to complete the 5' end to make complete clones. Twelve clones were sequenced. These twelve clones were identified as either "A", "B" or "C" as denoted by the C-terminus of the predicted amino acid sequence.

### Polymerase Chain Reaction.

The original PCR primer was based on the 5' end and the 3' end of a 416 base pair sequence having GenBank Database Accession No. T73849. This sequence was selected on the basis of a known motif present in cytokine receptors, "WSXWS".

The 5' primer had the sequence 73-96 of the 416 bp sequence. The 3' primer had the sequence 337-360 of the 416 bp sequence.

These primers were used to probe a human cDNA liver library (Stratagene). Standard methods were used.

This resulted in a PCR fragment having the sequence 73-360 of the 416 bp fragment.

### Hybridization.

The 300 bp PCR fragment was used to probe a human liver cDNA library (Stratagene) using standard methods. This second hybridization resulted in 13 positive clones. These were partial clones, incomplete at the 5' end.

### Completion of the 5' end.

Rapid Amplification of cDNA End ("RACE", kit, GIBCO/BRL) was used to obtain the full length clones.

### Sequencing results.

Sequencing revealed the three types of OB receptor DNAs. Of the thirteen clones, 4 clones were the "A" type (Seq. ID Nos. 1 and 2); 1 clone was the "B" type (Seq. ID Nos. 3 and 4) and 4 clones were of the "C" type (Seq. ID Nos. 5 and 6).

As can be seen from the Sequence Identifications (below), OB receptor A is 896 amino acids long, "B" is 904 amino acids long, and "C" is 958 amino acids long. These different OB receptors are identical at amino acid positions 1-891, and diverge almost completely beginning at position 892. The leader sequence is postulated to be, by hydrophobicity analysis, amino acids 1-21(M-A), 1-22 (M-F) or 1-28(M-I), with the mature protein beginning at positions 22(F), 23(N) or 29(T). Based on hydrophobicity analysis, the leader sequence is most likely to be at positions 1-21(M through A). Chinese Hamster Ovary Cell ("CHO") cell production of the secreted form of OB receptor protein also produced a protein having amino acid number 22 as the first amino acid of the mature protein. The transmembrane region is likely to begin at either position 840 (A) or 842(L) through position 862(I), 863(S) or 864(H). For OB receptor type "A", the last amino acid is located at position 896 and is a lysine (L). For OB receptor type "B", the last amino acid is located at position 904 and is a glutamine (Q). For OB receptor type "C", the last amino acid is located at position 958 and is glutamic acid (E).

For OB receptor protein type "C", the C-terminal region possesses high homology to a known human transposable element. From nucleotide 2737 through 2947 of the present human OB receptor protein type "C", there is a 98.1% homology with a 211 base section of a human retrotransposable element described in Ono et al., Nucl. Acids Res. 15: 8725-8737 (1987) (bases 520 through 731, SINE-R11, GENBANK accession no. x07417).

### EXAMPLE 2: TISSUE DISTRIBUTION

Tissue distribution was ascertained using two methods. The first method involved using the entire type "A" OB receptor. The second method involved using probes which are specific to the C-terminal region of the protein. Since these C terminal regions are divergent, the second method detected the tissue distribution of the different members of the OB receptor family.

The first method used a Northern Blot kit (Clontech), using the entire type A OB receptor DNA as a probe. The second method used PCR with primers specific to the nucleic acids encoding the divergent C terminus of the three types. Standard methods were used.

Table 2 shows the results for the Northern Blot and the PCR methods. The "+" indicates the investigator's subjective determination of the strength of signal. For the Northern Blot analysis, a triple "+++" indicates that a result (a dark "band" on the X-ray film) was seen upon overnight exposure of the film. A double "++" indicates that bands were seen at two weeks of exposure. A single "+" indicates that the bands were seen after three weeks of exposure. In addition, using this method, two molecular weights were observed, one at 4 Kb and one at 6.2 Kb. Although distribution was ubiquitous, the strongest signals were seen for ovary, heart and liver. For the PCR analysis, OB receptor "A" was seen in all tissue types tested (prostate, ovary, small intestine, heart, lung, liver and skeletal muscle), type "B" was seen only in lung and liver, and type "C" was seen in ovary, heart, lung and liver.

**Table 2**

| **Tissue Distribution of the Novel OB Receptor** | | | | | | |
|---|---|---|---|---|---|---|
| | Northern Blot | | | PCR | | |
| | 4 Kb | 6.2 Kb | | A | B | C |
| Spleen | - | + | | | | |
| Thymus | - | + | | | | |
| Prostate | - | + | | + | - | - |
| Testis | - | + | | | | |
| Ovary | - | +++ | | + | - | + |
| Small Intestine | - | ++ | | + | - | - |
| Colon | - | - | | | | |
| Peripheral blood Leukocyte | - | - | | | | |
| Heart | - | +++ | | + | - | + |
| Brain | - | - | | | | |
| Placenta | - | + | | | | |
| Lung | + | ++ | | + | + | + |
| Liver | +++ | +++ | | + | + | + |
| Skeletal Muscle | - | ++ | | + | - | - |
| Kidney | - | ++ | | | | |
| Pancreas | - | + | | | | |

### EXAMPLE 3: IDENTIFICATION OF HUMAN OB RECEPTOR GENOMIC DNA AND CHROMOSOME LOCALIZATION; IDENTIFICATION OF HUMAN OB RECEPTOR "D"

The full length human OB receptor genomic DNA was also prepared. OB receptor "A" cDNA, in its entirety, was used as a probe against a human genomic DNA library, using materials and methods from a commercially available kit (Genome Systems, using a human genomic library in a P1 vector). A single positive clone was detected. There are introns located at (with respect to OB receptor "A" DNA) base pair number: 559, 1059, 1350, 1667, 1817, 1937, 2060, 2277, 2460, 2662, and 2738.

The human OB receptor gene was localized to human chromosome 1P31 by FISH analysis (Genome Systems). Human chromosome 1 is thought to correspond to mouse chromosome 4C7, which is presumed to be the location of the db locus.

A further chromosomal sequence was isolated. This chromosomal DNA sequence was isolated from a human genomic library as described above. This chromosomal sequence encodes what is here denominated human OB receptor "D", and the encoded amino acid sequence is set forth in SEQ. ID No. 7. A cDNA encoding this amino acid sequence is set forth in SEQ. ID No. 8. The chromosomal DNA intron/exon junction map is set forth as SEQ. ID No. 9.

As with forms "A", "B", and "C", for the present form "D" OB receptor protein, the first amino acid of the mature protein is likely (using hydrophobicity analysis) to begin at position 22 (F), 23 (N) or 29 (T). The last amino acid of the protein is at position 1165 and is a valine residue. As with the other forms, the extracellular domain extends from position 22 (F), 23 (N) or 29 (T) to position 839 (D) or 841 (G). The transmembrane domain appears to begin at position 840 (A) or 842 (L). The end of the transmembrane domain appears to be located at position 862 (I), 863 (S) or 864 (H). The C-terminal region, beyond the transmembrane region, is likely to be involved in signal transduction, and is located at position 863 (S), 864 (H) or 865 (Q) through position 1165 (V).

The present OB receptor form "D" is identical to that published by Tartaglia et al, Cell 83: 1263-1271 (December 29, 1995) with the exception of a single amino acid change at amino acid position 976 (nucleotide codon begining at position 3022). The present type "D" amino acid at position 976 is aspartic acid, and the published amino acid corresponding to the same position is alanine. This is a non-conservative substitution, see infra, and since the location of the substitution is within a region thought important for signal transduction, this change could affect the function of the molecule.

### EXAMPLE 4: PREPARATION OF SOLUBLE OB RECEPTOR

Three forms of soluble human OB receptor have been prepared:
1. Leader + Extracellular Domain (Seq. ID Nos. 10 and 11): A recombinant form of the soluble human OB receptor was prepared. This form encompasses, in the immature protein, the leader sequence and the extracellular domain (amino acids 1-839). The mature protein would have the leader sequence deleted, and the first amino acid of the mature recombinant soluble human OB receptor would be 22 (F), 23 (N) or 29 (T). This protein was expressed as described below.
2. Leader + Extracellular Domain + C-terminal FLAG (Seq. ID No. 12): A second form of the recombinant soluble human OB receptor was also prepared. This form had a "FLAG" tag located at the "C" terminus of the protein. The "FLAG" peptide is a useful research tool as it allows one to follow the protein using an antibody which recognizes the "FLAG" peptide. Such reagents are commercially available (IBI, New Haven, CT). This protein was expressed as described below.
3. Native Splice Variant (Seq. ID Nos. 13 and 14): This form is believed to the the recombinant form of a naturally occurring secreted, soluble human OB receptor. This form has most of the amino acids found in the extracellular domain (amino acids 22-798), and a unique 6 amino acid sequence at the carboxyl terminus. Beginning at amino acid position 799 of Seq. ID No. 13, the amino acid sequence of this native splice variant human OB receptor protein is "G K F T I L."

### EXAMPLE 5: PREPARATION OF EXPRESSION VECTORS

Recombinant human OB receptor expression vectors have been prepared for expression in mammalian cells. As indicated above, expression may also be in non-mammalian cells, such as bacterial cells. The type "A" cDNA (Seq. ID No. 2) was placed into a commercially available mammalian vector (pCEP4, Invitrogen) for expression in mammalian cells, including the commercially available human embryonic kidney cell line, "293".

Recombinant human OB receptor expression vectors have been prepared for expression of recombinant soluble OB receptor, consisting of the leader sequence and the extracellular domain (Seq. ID Nos. 10 and 11), using the same system as above (the commercialy available mammalian vector pCEP4, and "293" cells). This recombinant soluble human OB receptor was also expressed in CHO cells in a similar way.

The "FLAG-tagged" form (Seq. ID No. 12) of the recombinant soluble human OB receptor, and the "D" form (Seq. ID No. 7) were also expressed in "293" cells in a similar fashion as above.

Detection of desired protein was accomplished using BIACORE (Pharmacia) analysis. This analysis is analogous to that described in Bartley et al., Nature 368: 558-560 (1994).

Essentially, the BIACORE machine measures affinity interactions between two proteins. In this case, the OB protein was immobilized on the machine, and conditioned media from cell lines expressing the OB receptor was added to the machine. Any receptor protein present in the conditioned media bound to the OB protein surface. The BIACORE machine gave a read-out indicating that receptor protein was being expressed. For recombinant soluble receptor (Seq. ID No. 10) expression in "293" cells, the read-out was 191.0 relative to a baseline readout of 0. For recombinant soluble receptor (SEq. ID No. 10) expression in CHO cells, the read-out was 150.9 relative to a baseline readout of 0. For recombinant soluble receptor with a C-terminal FLAG-tag (Seq. ID. No. 12), the read-out was 172.0 relative to a baseline of 0.

For expression in bacterial cells, one would typically eliminate that portion encoding the leader sequence (e.g., potentially amino acids 1-21, 1-22 or 1-28). One may add an additional methionyl at the N-terminus for bacterial expression. Additionally, one may substitute the native leader sequence with a different leader sequence, or other sequence for cleavage for ease of expression.

### EXAMPLE 6: DEMONSTRATION OF SIGNAL TRANSDUCTION

This example demonstrates that the "D" form is active to produce a signal within a cell, whereas in the same cell type, the "A" form does not. The signal transduction assay was performed by the use of "293" cells transiently expressing either the "A" or the "D" form (see above for preparation of the "293" expression clones). Phosphorylation of molecules predicted to be involved in signal transduction within the cell was examined upon OB protein binding to the OB receptor protein tested. The results demonstrate that upon binding of OB protein to the extracellular domain, the "D" form of the present OB protein receptor transduces a signal sufficient to initiate phosphorylation of signalling molecules.

### Methods

1. OB receptor molecules. As indicated above, the "A" form (Seq. ID No. 1) and the "D" form (Seq. ID. No. 7) were studied.
2. Expression system. The pCEP 4 system (as described above) having inserted DNA encoding the "A" form (Seq. ID No. 2) or the "D" form (Seq. ID No. 8) was used to transfect "293" cells. These cells did not allow for the pCEP4 vector to integrate into the genome, so such expression was transient. Non-recombinant (mock-transfected) cells were also prepared as controls.
3. Detection of phosphorylation. Mock transfected cells and cells expressing the "A" form or the "D" form were analyzed. Prior to treatment the cells were serum-starved by incubation in media with 0.5% serum for 16 hours prior to the treatments. The cells were treated with the OB protein (10 mg/ml) for 15 minutes at 37°C, after which the cells were lysed in modified NP40 buffer (50 mM Tris, pH 8.0, 150 mM sodium chloride, 1% NP40, 10 mg/ml aprotinin, 5mM EDTA, 200 mM sodium orthovanadate). Phosphotyrosine containing proteins were immunoprecipitated (Anti-phosphotyrosine antibody 4G10, UBI, Lake Placid, NY), and separated by SDS polyacrylamide gel electrophoresis. After electrophoresis and electroblotting to membranes the immunoprecipitates were probed with antibodies to various signal transduction molecules. Antibodies to STATs, JAKs and ERKs were purchased from Santa Cruz Biotechnology Inc. Immune complexes were detected by horseradish peroxidase conjugated secondary reagents using chemiluminescence as described by the manufacturer (ECL, Amersham). As a positive control, 32D cells were treated with IL-3, which is known to activate by tyrosine phosphorylation most of the molecules being analyzed.
4. Results. Results are presented in Table 5 3, below. As can be seen, only the "D" form was able to respond to either mouse or human OB protein as detected by phosphorylation of JAK and STAT molecules. A "+" designation indicates signal was detected, a "-" designation means that no signal was observed.

**TABLE 3**

| Signal /AB‡ | 293 Alone | 293/D hrOB* | 293/D mrOB** | 293/A hrOB# | 293/A mrOB## | 32D IL-3 |
|---|---|---|---|---|---|---|
| STAT1 | - | + | | | | |
| STAT3 | - | + | + | - | - | + |
| STAT5 | - | + | + | | | + |
| JAK1 | - | + | + | - | - | + |
| JAK2 | - | + | + | - | - | + |
| JAK3 | - | - | - | | | - |
| TYK2 | - | + | + | | | - |
| ERKs 1, 2 | - | - | - | - | - | + |

| | | | | | | |
|---|---|---|---|---|---|---|
| ‡ Antibody detection target | | | | | | |
| * 293 cells expressing receptor form "D", treated with recombinant human OB | | | | | | |
| ** 293 cells expressing receptor form "D" treated with recombinant murine OB | | | | | | |
| # 293 cells expressing receptor form "A" treated with recombinant human OB | | | | | | |
| ## 293 cells expressing receptor form "A" treated with recombinant murine OB | | | | | | |

The "D" form is capable of initiating signalling through the JAK/STAT pathways in 293 cells, whereas the "A" form cannot.

### EXAMPLE 7: USE OF SOLUBLE OB RECEPTOR AS A THERAPEUTIC

This example demonstrates that soluble OB receptor protein acts to protect the activity of OB protein. Below, soluble OB receptor and/or OB protein was delivered to a mammal via "gene transplant" -- that is, via bone marrow cells engineered to express the desired DNAs. When soluble OB receptor combined with OB protein was delivered, the animals lost more weight than delivery of OB protein alone. This demonstrates the protective activity of OB receptor protein.

While not wishing to be bound by theory, one explanation of the mode of action is that soluble OB receptor protein acts to protect the OB protein in serum from agents or conditions which could diminish its activity. The protective action appears to increase circulating half-life of the protein. As such, the present example demonstrates that OB receptor either alone, or administered as a complex with OB protein (or analog or derivative thereof) could act as a therapeutic agent.

### Materials and methods:

### 1. Preparation of recombinant ob retroviral vector Packaging Cells.

Use of murine ob cDNA. Full length wild-type murine *ob* cDNA was amplified by the PCR using synthetic oligonucleotides designed from the published sequence Zhang et al., Nature *372*: 425-432 (1994).Linkers (An Eco RI linker and a Bgl II linker) were used to facilitate subcloning.

Use of soluble recombinant human OB receptor cDNA. Methods similar to those above were used. A construct containing the recombinant human soluble receptor of Seq. ID No. 10 was used, and modified with linkers to facilitate cloning (i.e., the addition of a Bgl II restriction endonuclease recognition site).

Placement of desired cDNA into vector. PCR products were digested with EcoRI and *Bgl*II and cloned into similarly-digested parental vector (pMSCV2.1) under the transcriptional control of the viral LTR promoter. The parental MSCV vector (supplied by R. Hawley, University of Toronto, Canada) was derived from MESV (murine embryonic stem cell virus) and contains a neomycin phosphotransferase resistance (neo^{r}) gene driven by an internal mouse phosphoglycerate kinase (PGK) promoter, as described· Hawley, et al, J. Exp. Med. 176: 1149 -1163 (1992). The parental plasmid pMSCV2.1 and pMSCV-OB were independently electroporated into the GP+E-86 packaging cell line (supplied by Dr. A. Bank, Columbia University, NY) Markowitz et al., J. Virol. 62:1120-1124 (1988). Transient supernatants were harvested from electroporated populations and used to infect tunicamycin treated parental GP+E-86 cells. Tunicamycin treatment relieves the block to superinfection of the parental packaging cells. G418 (0.78 mg/mL, 67% active, GIBCO Laboratories, Life Technologies, Inc., Grand Island, NY) resistant clones were selected from each infected population and titered by infection of NIH3T3 cells. Clones with the highest G418 resistant titer were expanded and frozen as aliquots. Each bone marrow infection and transplantation experiment used aliquots from the same passage of frozen viral packaging cells. Both the parental and *ob* packaging cell lines were tested for the presence of, and found to be free from, replication competent virus using a sensitive marker rescue assay. Moore, et al., (1993) in: Gene Targeting: A Practical Approach, Joyner, Ed. (Oxford University Press, New York, NY).

### 2. Production of Retroviral Supernatants.

Recombinant virus-producing packaging cell lines were grown in 175cm² tissue culture flasks in Iscove's Modified Dulbecco's Medium (IMDM) (GIBCO), 10% (v/v) FBS, at 37°C. Sub-confluent (approximately 60%) monolayers of cells were fed with fresh medium 24h prior to harvest of virus-containing supernatants. Viral supernatants were removed from packaging cell lines by aspiration, sterile filtered (0.45mM) and added directly to bone marrow cultures. Fresh aliquots of frozen packaging cell lines were thawed for use in each experiment.

### 3. Bone Marrow Infection and Transplantation.

Eight to 12-week old female C57BL/6J (+/+) or (*ob*/*ob*) mice were used as bone marrow donors and recipients. All mice were purchased from The Jackson Laboratory (Bar Harbor, ME) and housed under specific pathogen-free conditions in a vivarium in accordance with governmental regulations and institutional guidelines.

Bone marrow cells were harvested from femurs and tibias of donor mice 4 days post 5-fluorouracil (5-FU, Sigma Chemical Co., St. Louis, MO) treatment (150 mg/kg i.v.). Bone marrow cells (6 X 10⁵/mL) were incubated in 150mm tissue culture dishes (30mL/dish) containing fresh viral supernatant (as described above), 15% FBS, 6 mg/mL polybrene (Sigma), 0.1% bovine serum albumin (BSA, Fraction V, Sigma), 2.5 ng/mL recombinant mouse IL-3 (rmIL-3), 100 ng/mL each of recombinant human IL-6 (rhIL-6), recombinant human IL-11 (rhIL-11), and recombinant rat SCF (rrSCF). All growth factors were produced by Amgen, Inc. (Thousand Oaks, CA). Culture media were replaced daily for 3 days with fresh virus-containing supernatant and growth factors.

At the end of the infection period, total non-adherent and adherent cells were washed and resuspended in 1% BSA-saline and transplanted into g-irradiated (12 Gy, Cs¹³⁷) mice. Each animal was transplanted with 2.5 X 10⁶ syngeneic cells. There were approximately 10 animals per cohort.

### 4. Analysis of OB protein expression in transfected cells and transplanted animals.

For transfected bone marrow cells, Western analysis was performed. Vector packaging cell supernatant was resolved by SDS-PAGE (16% acrylamide), then transferred to Hybond-ECL (Amersham, Arlington Heights, IL). The filter was incubated with affinity-purified rabbit a-mouse OB protein polyclonal antibody (1mg/mL) in T-TBS buffer (20mM Tris-chloride, pH7.6, 137mM NaCl, 0.1% Tween20) at room temperature for 45 min. Horseradish peroxidase (HRP)-conjugated donkey a-rabbit IgG (Amersham) was diluted in T-TBS (1:2500) and incubated with the filter at room temperature for 45 min. Enhanced chemiluminescence (ECL, Amersham) detection was performed as recommended by the manufacturer.

For transplanted animals, serum was analyzed. Animals were bled retroorbitally, under isofluorane anesthesia. Serum from transplanted *ob*/*ob* animals was resolved by SDS-PAGE (4-20% acrylamide) under nonreducing and reducing conditions, then transferred to Trans-Blot (Bio-Rad Laboratories, Hercules, CA) membranes. The membranes were incubated for 2 hours at room temperature with HRP-conjugated rabbit a-mouse OB protein antibody (0.125mg/mL) in T-TBS buffer containing 5% fetal bovine serum and 1% bovine serum albumin. Bound OB protein was detected by ECL (Amersham), performed as recommended by the manufacturer.

For quantitation of soluble OB protein levels, serum from transplanted animals was subjected to ELISA analysis. Briefly, affinity-purified rabbit a-OB protein polyclonal antibody was coated onto 96-well plates. Standards (purified recombinant 0B protein monomer, Pelleymounter et al., Science 269: 540-543 (1995) and experimental samples were added, and the plates were incubated at room temperature. The plates were washed twice and affinity-purified rabbit a-OB protein antibody conjugated to horseradish peroxidase was added. Following incubation at room temperature, the plates were washed four times with TNE-Tween20. TMB/peroxide substrate was added and the color reaction was read at 450nm in a Molecular Devices plate reader. OB protein concentrations in sera were estimated by comparison to a standard curve prepared from internal standards. OB protein levels were reliably measured in samples containing >160 pg/mL.

### 5. Body Weight and Food Intake .

Mice were offered pelletized rodent chow (PMI Feeds, Inc., St. Louis, MO) ad libitum. The body weight of individual animals was measured daily for the first two months of analysis, and weekly thereafter. Food consumption was measured daily on selected groups of individually-housed animals.

### Results

Results are presented in Tables 4 and 5 below. Administration of OB protein receptor increased the effectiveness of OB protein. This may have been accomplished via an increased circulation time of OB protein in the presence of OB protein receptor.

As can be seen in the Table, animals administered a combination of OB protein and OB protein receptor (via genetic therapy) had a greater weight loss after 28 days than either composition alone. The Table presents the results of two experiments ("_/_"). As can be seen, use of the OB protein alone at day 40 resulted in animals with 87.5% and 72.2% of the starting weight. Using OB receptor in combination with OB protein, however, resulted in animals with 68% and 53.6% of the starting weight. Use of the receptor alone appeared to have little effect, if any.

**TABLE 4**

| Treatment | Weight(g) decrease at day 28 (ave) | % starting weight (ave) day 28 | % starting weight (ave) day 40 |
|---|---|---|---|
| OB alone* | 6.3/12.7 | 87.9/75.3 | 87.5/72.2 |
| Receptor** alone | [1.4]/[0.3] | 103/100.6 | 104.2/101.7 |
| OB + Receptor*** | 12.6/16.8 | 76.3/67.5 | 68/53.6 |

| | | | |
|---|---|---|---|
| * 50% bone marrow cells transfected with OB protein cDNA as described above, and 50% bone marrow cells without genetic alteration | | | |
| ** 50% bone marrow cells transfected with OB receptor protein cDNA as described above, and 50% bone marrow cells without genetic alteration | | | |
| *** 50% bone marrow cells transfected with OB protein cDNA as described above, and 50% bone marrow cells transfected with OB receptor protein cDNA as described above. | | | |

Table 5, below, contains results of the OB levels found in the serum from animals administered OB protein alone, or administered OB protein in combination with OB protein receptor (via the "gene therapy" method of this example). The data reflect nanograms of OB protein per milliliter of serum, plus or minus the standard error of the mean.

**TABLE 5**

| Treatment | Experiment #1‡ | Experiment #2‡‡ |
|---|---|---|
| OB alone* | 2.93 +/- 0.77 | 9.74 +/- 1.02 |
| Receptor** alone | 0.08 +/- 0.05 | 0.12 +/- 0.07 |
| OB + Receptor*** | 12.11 +/- 1.90 | 15.18 +/- 2.52 |

| | | |
|---|---|---|
| * 50% bone marrow cells transfected with OB protein cDNA as described above, and 50% bone marrow cells without genetic alteration | | |
| ** 50% bone marrow cells transfected with OB receptor protein cDNA as described above, and 50% bone marrow cells without genetic alteration | | |
| *** 50% bone marrow cells transfected with OB protein cDNA as described above, and 50% bone marrow cells transfected with OB receptor protein cDNA as described above. | | |
| ‡ Experiment #1 was conducted as described above, with OB protein serum levels measured after 38 days. | | |
| ‡‡ Experiment #2 was also conducted as described above, with OB protein serum levels measured after 24 days. | | |

The data demonstrate the protective effects of OB receptor. As can be seen, in the presence of OB receptor, OB protein has a higher accumulation in the serum. The degree of accumulation is observed to increase inversely with the levels of OB protein in the serum. In Experiment #1 (with a base OB protein level of about 2.93 ng/ml), the OB protein serum level increased about 400% with the addition of receptor, where in Experiment #2 (with a base of about 9.74), the OB protein serum level increased by about 25%.

OB receptor administered either alone or in association with OB protein (or analogs or derivatives thereof) may serve to increase the circulation time of OB protein, and therefore enhance the therapeutic efficacy of either exogenous or endogenous OB protein.

### EXAMPLE 8: PREPARATION OF SELECTIVE BINDING MOLECULES

Animals were immunized for the preparation of polyclonal antibodies using the following peptides (with respect to the numbering of the amino acids for OB receptor A, Seq. ID No. 1): 54-64; 91-100; 310-325; 397-406; 482-496; 874-885; and, with respect to amino acids of OB receptor "C" (Seq. ID No. 5), 910-929. Some of the polyclonal antibodies prepared (in rabbits) were tested for ability to bind to recombinant human OB receptor protein. The polyclonal antibody prepared against amino acids 54-64 was found to have the highest affinity for recombinant human OB receptor protein. The polyclonal antibody prepared against amino acids 397-406 was also found to bind to recombinant human OB receptor protein. The polyclonal antibody prepared against amino acids 91-100 was found to slightly bind to recombinant human OB receptor protein. The polyclonal antibody prepared against amino acids 874-885 was found not to bind to recombinant human OB receptor protein.

An additional study was performed which demonstrates the expression and purification of the extracellular domain of the OB receptor protein in CHO cells, and antibodies which recognize this OB protein receptor extracellular domain.

The extracellular domain of the human OB receptor protein was expressed as a secreted, soluble protein in CHO cells as previously described supra*.* Individual cell lines were isolated and grown in increasing amounts of methotrexate to increase selection/expression of the recombinant receptor protein (100, 200 or 500 micrograms methotrexate per ml of media). Conditioned media from the CHO cell lines was collected, and the proteins in the conditioned media were fractionated by SDS-PAGE. The OB receptor extracellular domain migrated as a broad band with an apparent size range of about 140 kDa to about 200 kDa. The OB receptor protein extracellular domain was detected by Western Blot analysis using polyclonal antibodies prepared against a portion of the extracellular domain of the OB receptor protein. The unfolded, bacterially expressed protein was used as an antigen to generate antisera in rabbits. The identified OB receptor extracellular domain was purified by affinity chromatography. The purified protein was sequenced at the amino terminus to confirm that it was the OB receptor and also to determine the start of the mature protein (after signal peptide cleavage) as expresed in CHO cells. It was found that amino acid no. 22 (according to the amino acid sequence numbering of Seq. ID No. 1, infra), was the first amino acid of the mature protein as expressed in CHO cells.

Other immunogenic peptides may be used. Polyclonal, monospecific polyclonal, monoclonal, antibody fragments, and recombinant antibodies may be prepared using methods available to those skilled in the art.

One may further use recombinant techniques or peptide synthesis methods to alter the character of such selective binding molecules. This may be accomplished by preparing recombinant antibodies having altered complementarity determining regions (sometimes referred to in the art as "CDR's") to, for example "humanize" the antibodies by using human F_{c} (constant) regions. Other types of recombinant antibodies, for example, those having CDR's altered to enhance affinity or selectivity to one or more members of the OB receptor family, may be prepared and used using methods available to those skilled in the art. See Winter et al., Nature 349: 293-299 (1991).

The present OB receptor protein may be used as an assay to screen for desired selective binding molecules. Such assay may be based on binding capability, or biological activity, or, other means of detecting signal transduction. For example, if one were to prepare a series of modified antibodies, one could test them for affinity (i.e, binding strength) against the target OB receptor.

The selective binding molecules may be useful for diagnostic purposes, such as tissue distribution analysis, or to diagnose the relative affinity of an individual's OB receptors for such selective binding molecule to determine the functionality of an individual's OB receptor during a course of therapy. Selective binding molecules may be alternative therapeutic or cosmetic products to OB protein.

### EXAMPLE 9: GENE THERAPY

One may deliver the present OB receptor protein via gene therapy, as described infra.

One may envision, using materials and methods available to those skilled in the art and provided herein, using T-cells as an agent carrying DNA expressing OB receptor for gene therapy. An individual would have T-cells selected using CD34+ selection and a magnetic microparticles selection device. Such cells would be transfected with the desired DNA, or the regulation of the desired coding region may be altered using homologous recombination or other in situ techniques. The transduced cells could be selected empirically, using means to detect the desired protein, or a marker may be included which permits indirect detection (i.e., a selectable marker as is known in the art). Optionally, such cells could be expanded, for example, using one or more growth factors such as SCF or an interleukin, and such cells could be stored for future use. In such a way, the procedure would only have to be accomplished once or infrequently in an individual's lifetime, for later transfer into the individual. The cells would be re-planted into the individual, and the individual would be monitored for desired therapeutic effect, such as weight loss/maintenance of weight, diabetes recurrence, blood lipid levels, or other conditions.

### Illustrative Nucleic Acid and Amino Acid Sequences

The below amino acid and DNA sequences are those to which reference has been made. An asterick("*") indicates the position of a stop codon.

### Human OB Receptor "A" Amino Acid Sequence (Seq. ID No. 1 (Amino Acid, single letter abbreviation):

### Human OB Receptor "A" DNA Sequence (Seg, ID No. 2 (DNA)):

### Human OB Receptor "B" Amino Acid Sequence (Seq. ID No. 3 (Amino Acid)):

### Human OB Receptor "B" DNA Sequence (Seq. ID No. 4 (DNA)):

### Human OB Receptor "C" Amino Acid Sequence (Seq. ID No. 5 (Amino Acid)):

### Human OB Receptor "C" DNA Sequence (Seq. ID No. 6 (DNA)):

### Human OB Receptor "D" Amino Acid Sequence (Sequence ID No. 7)

### Human OB Receptor "D" Nucleic Acid Sequence(Sequence ID No.8)

### Human OB Receptor Protein "D" Chromosomal DNA (Seg. ID No. 9)

### Human OB Receptor Protein, Recombinant Secreted Receptor amino acid sequence (Seq. ID. No. 10):

### Human OB Receptor Protein, Recombinant Secreted Receptor DNA sequence (Seq. ID. No. 11):

### Human OB Receptor Protein, Recombinant secreted Receptor DNA sequence with C-terminal FLAG (Seq. ID. No. 12):

### Recombinant Human OB Receptor Protein, Natural Splice Variant amino acid sequence (Seq. ID. No. 13)

### Human OB Receptor Protein, Natural Splice Variant DNA (Seq. ID No. 14)

While the present invention has been described in terms of preferred embodiments, it is understood that variations and modifications will occur to those skilled in the art. Therefore, it is intended that the appended claims cover all such equivalent variations which come within the scope of the invention as claimed.

## Claims

1. An OB receptor protein preparation containing an OB receptor protein, optionally in a pharmaceutically acceptable formulation, wherein said OB receptor protein consists of the amino acid sequence selected from among amino acid sequences (according to Seq. ID No.1):
(a) 1-896;
(b) 22-896 optionally with an N-terminal methionyl residue;
(c) 23-896 optionally with an N-terminal methionyl residue;
(d) 29-896 optionally with an N-terminal methionyl residue;
(e) 22-891 optionally with an N-terminal methionyl residue;
(f) 23-891 optionally with an N-terminal methionyl residue;
(g) 29-891 optionally with an N-terminal methionyl residue;
(h) sequences (e) through (g) further having the C-terminal amino acids, beginning at position 892, of OB receptor B (Seq. ID No. 3) or C (Seq. ID. No. 5); and
(i) a chemically modified derivative of any of subparts (a) through (h).

2. An OB receptor protein preparation of Claim 1 wherein said OB receptor protein has substituted the C-terminal amino acids, beginning at position 799, by G K F T I L (Seq. ID No. 13).

3. A OB receptor protein preparation according to Claim 1 or 2, wherein the extracellular domain, which extends from position 22(F), 23(N) or 29(T) to position 839(D) or 841(G) of said OB receptor protein is modified, said modification selected from among:
(a) deletion of all or part of the random coil domain from about amino acid 642 to amino acid 839 or 841,
(b) modification of one or both "WSXWS" boxes by substitution of the first serine with another amino acid;
(c) modification of one or both "WSXWS" boxes by substitution of the last serine with another amino acid; and
(d) modification of one or both "WSXWS" boxes by substitution of the first tryptophan with another amino acid; and

4. A DNA molecule encoding an OB receptor protein according to any of Claims 1-3.

5. An expression or cloning vector containing a DNA of Claim 4.

6. A procaryotic or eucaryotic host cell containing the vector of Claim 5.

7. A host cell of Claim 6 which is an isolated human host cell.

8. A process for producing an OB receptor protein comprised of culturing, under suitable conditions, a host cell according to Claim 6, obtaining the OB receptor produced, and optionally preparing a pharmaceutical composition containing said OB receptor.

9. Use of an OB receptor protein according to Claims 1-3, or produced by the process of Claim 8, for manufacturing a medicament for the treatment of obesity, diabetes, high blood lipid levels, or high cholesterol levels.

10. An OB protein/OB receptor protein complex preparation, containing an OB protein moiety and an OB receptor protein moiety, optionally in a pharmaceutically acceptable formulation, wherein:
(a) said OB receptor protein is selected from among those set forth in any of Claims 1 to 3; and
(b) said OB protein moiety is selected from among:
(i) a naturally occurring OB protein; and,
(ii) a non-naturally occurring OB protein, analog or derivative thereof.

11. Use of am OB protein/OB receptor protein complex preparation according to Claim 10, for manufacturing a medicament for the treatment of obesity, diabetes, high blood lipid levels, or high cholesterol levels.

## Patentansprüche

1. OB-Rezeptorprotein-Zubereitung, die ein OB-Rezeptorprotein enthält, wahlweise in einer pharmazeutisch vertretbaren Formulierung, wobei das OB-Rezeptorprotein aus der Aminosäuresequenz besteht, die aus folgenden Aminosäuresequenzen ausgewählt ist (gemäß SEQ ID No. 1):
(a) 1-896;
(b) 22-896, wahlweise mit einem N-terminalen Methioninrest;
(c) 23-896, wahlweise mit einem N-terminalen Methioninrest;
(d) 29-896, wahlweise mit einem N-terminalen Methioninrest;
(e) 22-891, wahlweise mit einem N-terminalen Methioninrest;
(f) 23-891, wahlweise mit einem N-terminalen Methioninrest;
(g) 29-891, wahlweise mit einem N-terminalen Methioninrest;
(h) wobei die Sequenzen (e) bis (g) weiterhin die C-terminalen Aminosäuren, beginnend in Position 892, eines OB-Rezeptors B (SEQ ID No. 3) oder C (SEQ ID No. 5) aufweisen; und
(i) ein chemisch modifiziertes Derivat von jeder der in (a) bis (h) aufgeführten Sequenzen.

2. OB-Rezeptorprotein-Zubereitung nach Anspruch 1, wobei die C-terminalen Aminosäuren des OB-Rezeptorproteins, beginnend in Position 799 durch GKFTIL (SEQ ID No. 13) substituiert sind.

3. OB-Rezeptorprotein-Zubereitung nach Anspruch 1 oder 2, wobei die extrazelluläre Domäne, die sich von Position 22(F), 23(N) oder 29(T) bis Position 839(D) oder 841(G) erstreckt, des OB-Rezeptorproteins modifiziert ist, wobei die Modifikation aus folgenden ausgewählt ist:
(a) Deletion der gesamten oder eines Teils der Zufallsknäuel-Domäne von ungefähr Aminosäure 642 bis Aminosäure 839 oder 841;
(b) Modifikation von einer der oder beiden "WSXWS"-Boxen durch Substitution des ersten Serins durch eine andere Aminosäure;
(c) Modifikation von einer der oder beiden "WSXWS"-Boxen durch Substitution des letzten Serins durch eine andere Aminosäure; und
(d) Modifikation von einer der oder beiden "WSXWS"-Boxen durch Substitution des ersten Tryptophans durch eine andere Aminosäure.

4. DNA-Molekül, das ein OB-Rezeptorprotein nach einem der Ansprüche 1 bis 3 kodiert.

5. Expressions- oder Klonierungsvektor, der eine DNA nach Anspruch 4 enthält.

6. Eine prokaryotische oder eukaryotische Wirtszelle, die den Vektor nach Anspruch 5 enthält.

7. Wirtszelle nach Anspruch 6, die eine isolierte menschliche Wirtszelle ist.

8. Verfahren zum Herstellen eines OB-Rezeptor-Proteins, die ein Kultivieren unter geeigneten Bedingungen, eine Wirtszelle nach Anspruch 6, ein Erhalten des hergestellten OB-Rezeptors und wahlweise ein Herstellen einer pharmazeutischen Zubereitung, die den OB-Rezeptor enthält, umfaßt.

9. Verwendung eines OB-Rezeptorproteins nach den Ansprüchen 1 bis 3 oder hergestellt durch das Verfahren nach Anspruch 8 zum Herstellen eines Medikaments zur Behandlung von Fettleibigkeit, Diabetes, hohen Blutfettspiegeln oder hohen Cholesterinspiegeln.

10. OB-Protein/OB-Rezeptorprotein-Komplex-Zubereitung, die einen OB-Protein-Rest und einen OB-Rezeptorprotein-Rest enthält, wahlweise in einer pharmazeutisch vertretbaren Formulierung, wobei:
(a) das OB-Rezeptorprotein ausgewählt ist aus jenen, die in einem der Ansprüche 1 bis 3 dargelegt sind; und
(b) der OB-Protein-Rest ausgewählt ist aus folgendem:
(i) einem natürlich vorkommenden OB-Protein; und
(ii) einem nicht natürlich vorkommenden OB-Protein, einem Analogon oder Derivat davon.

11. Verwendung einer OB-Protein/OB-Rezeptorprotein-Komplex-Zubereitung nach Anspruch 10 zur Herstellung eines Medikaments zur Behandlung von Fettleibigkeit, Diabetes, hohen Blutfettspiegeln oder hohen Cholesterinspiegeln.

## Revendications

1. Préparation de protéine de récepteur OB contenant une protéine de récepteur OB, éventuellement dans une formulation pharmaceutiquement acceptable, dans laquelle ladite protéine de récepteur OB se compose de la séquence d'acides aminés choisie parmi les séquences d'acides aminés (selon la Seq. ID N° 1) :
(a) 1-896 ;
(b) 22-896 éventuellement avec un résidu de méthionyle N-terminal ;
(c) 23-896 éventuellement avec un résidu de méthionyle N-terminal ;
(d) 29-896 éventuellement avec un résidu de méthionyle N-terminal ;
(e) 22-891 éventuellement avec un résidu de méthionyle N-terminal ;
(f) 23-891 éventuellement avec un résidu de méthionyle N-terminal ;
(g) 29-891 éventuellement avec un résidu de méthionyle N-terminal ;
(h) des séquences (e) à (g) ayant de plus les acides aminés C-terminaux, commençant en position 892, B (Seq. ID N° 3) ou C (Seq. ID N° 5) de récepteur OB ; et
(i) un dérivé modifié chimiquement d'une quelconque des sous-parties (a) à (h).

2. Préparation de protéine de récepteur OB de la revendication 1, dans laquelle ladite protéine de récepteur OB a remplacé les acides aminés C-terminaux, commençant en position 799, par G K F T I L (Seq. ID N° 13).

3. Préparation de protéine de récepteur OB selon la revendication 1 ou 2, dans laquelle le domaine extracellulaire, qui s'étend de la position 22(F), 23(N) ou 29(T) à la position 839 (D) ou 841 (G), de ladite protéine de récepteur OB, est modifié, ladite modification choisie étant parmi :
(a) une délétion de tout ou partie du domaine enroulé aléatoire d'environ l'acide aminé 642 à l'acide aminé 839 ou 841,
(b) une modification d'une ou des deux boîtes "WSXWS" par remplacement de la première sérine par un autre acide aminé ;
(c) une modification d'une ou des deux boîtes "WSXWS" par remplacement de la dernière sérine par un autre acide aminé ; et
(d) une modification d'une ou des deux boîtes "WSXWS" par remplacement du premier tryptophane par un autre acide aminé.

4. Molécule d'ADN codant une protéine de récepteur OB selon une quelconque des revendications 1-3.

5. Vecteur d'expression ou de clonage contenant un ADN de la revendication 4.

6. Cellule hôte procaryote ou eucaryote contenant le vecteur de la revendication 5.

7. Cellule hôte de la revendication 6 qui est une cellule hôte humaine isolée.

8. Procédé pour produire une protéine de récepteur OB consistant à mettre en culture, sous des conditions appropriées, une cellule hôte selon la revendication 6, obtenir le récepteur OB produit, et éventuellement préparer une composition pharmaceutique contenant ledit récepteur OB.

9. Utilisation d'une protéine de récepteur OB selon les revendications 1-3, ou produite par le procédé de la revendication 8, pour fabriquer un médicament pour le traitement de l'obésité, du diabète, de taux élevés de lipides sanguins ou de taux élevés de cholestérol.

10. Préparation de complexe protéine OB/protéine de récepteur OB, contenant une fraction de protéine OB et une fraction de protéine de récepteur OB, éventuellement dans une formulation pharmaceutiquement acceptable, dans laquelle :
(a) ladite protéine de récepteur OB est choisie parmi celle indiquée dans l'une quelconque des revendications 1 à 3 ; et
(b) ladite fraction de protéine OB est choisie parmi :
(i) une protéine OB existant naturellement ; et
(ii) une protéine OB existant non naturellement, analogue ou dérivé de celle-ci.

11. Utilisation d'une préparation de complexe protéine OB/protéine de récepteur OB selon la revendication 10, pour fabriquer un médicament pour le traitement de l'obésité, du diabète, de taux élevés de lipides sanguins, ou de taux élevés de cholestérol.
